(19) Europäisches Patentamt
European Patent Office
Office européen des brevets

(11) EP 3 647 869 A1

(12) # EUROPEAN PATENT APPLICATION
published in accordance with Art. 153(4) EPC

(43) Date of publication:
**06.05.2020 Bulletin 2020/19**

(21) Application number: **18825432.0**

(22) Date of filing: **25.06.2018**

(51) Int Cl.:
***G03F 7/004*** (2006.01) ***C07D 251/24*** (2006.01)
***C09D 7/63*** (2018.01) ***C09D 201/00*** (2006.01)
***G03F 7/023*** (2006.01) ***G03F 7/027*** (2006.01)
***G03F 7/032*** (2006.01) ***G03F 7/039*** (2006.01)
***G03F 7/11*** (2006.01) ***G03F 7/20*** (2006.01)
***G03F 7/26*** (2006.01) ***G03F 7/40*** (2006.01)
***H01L 21/027*** (2006.01)

(86) International application number:
**PCT/JP2018/024048**

(87) International publication number:
**WO 2019/004142 (03.01.2019 Gazette 2019/01)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.06.2017 JP 2017126543**

(71) Applicant: **Mitsubishi Gas Chemical Company, Inc.
Tokyo 100-8324 (JP)**

(72) Inventors:
- **MIKI, Yasushi
Hiratsuka-shi, Kanagawa 254-0016 (JP)**
- **ECHIGO, Masatoshi
Tokyo 100-8324 (JP)**

(74) Representative: **Müller-Boré & Partner Patentanwälte PartG mbB
Friedenheimer Brücke 21
80639 München (DE)**

(54) **FILM-FORMING MATERIAL, LITHOGRAPHIC FILM-FORMING COMPOSITION, OPTICAL COMPONENT-FORMING MATERIAL, RESIST COMPOSITION, RESIST PATTERN FORMATION METHOD, RESIST PERMANENT FILM, RADIATION-SENSITIVE COMPOSITION, AMORPHOUS FILM PRODUCTION METHOD, LITHOGRAPHIC UNDERLAYER FILM-FORMING MATERIAL, LITHOGRAPHIC UNDERLAYER FILM-FORMING COMPOSITION, LITHOGRAPHIC UNDERLAYER FILM PRODUCTION METHOD, AND CIRCUIT PATTERN FORMATION METHOD**

(57) Provided is a film-forming material; to which a wet process can be applied, which is useful for forming such as; a lithographic film having excellent heat resistance, resist pattern shape, etching resistance, embedding characteristics on a stepped substrate, and film flatness, and an optical component having excellent heat resistance, transparency, and refractive index, and which comprises a triazine-based compound represented by formula (1).

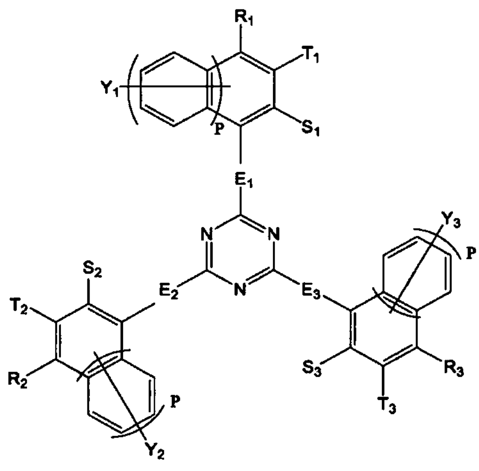

(1)

EP 3 647 869 A1

## Description

### Technical Field

The present invention relates to a film forming material, a composition for film formation for lithography, a material for optical component formation, a resist composition, a resist pattern formation method, a permanent film for a resist, a radiation-sensitive composition, a method for producing an amorphous film, an underlayer film forming material for lithography, a composition for underlayer film formation for lithography, a method for producing an underlayer film for lithography, and a circuit pattern formation method.

### Background Art

In the production of semiconductor devices, fine processing is practiced by lithography using photoresist materials. In recent years, further miniaturization based on pattern rules has been demanded along with an increase in the integration and speed of LSI. Further, lithography using light exposure, which is currently used as a general purpose technique, is approaching the limit of essential resolution derived from the wavelength of a light source.

The light source for lithography used upon forming resist patterns has been shifted to ArF excimer laser (193 nm) having a shorter wavelength from KrF excimer laser (248 nm). However, as the miniaturization of resist patterns proceeds, the problem of resolution or the problem of collapse of resist patterns after development arises. Therefore, resists have been desired to have a thinner film. Nonetheless, if resists merely have a thinner film, it is difficult to obtain the film thicknesses of resist patterns sufficient for substrate processing. Therefore, there has been grown a need for a process of preparing a resist underlayer film between a resist and a semiconductor substrate to be processed, and imparting functions as a mask for substrate processing to this resist underlayer film in addition to a resist pattern.

Various resist underlayer films for such a process are currently known. As a material for obtaining resist underlayer films for lithography having a selectivity of a dry etching rate close to that of resists, unlike conventional resist underlayer films having a fast etching rate, an underlayer film forming material for a multilayer resist process containing a resin component having at least a substituent that generates a sulfonic acid residue by eliminating a terminal group under application of predetermined energy, and a solvent has been suggested (see, for example, Patent Literature 1). As a material for obtaining such resist underlayer films for lithography having a selectivity of a dry etching rate smaller than that of resists, a resist underlayer film material comprising a polymer having a specific repeat unit has been suggested (see, for example, Patent Literature 2). As a material for obtaining such resist underlayer films for lithography having the selectivity of a dry etching rate smaller than that of semiconductor substrates, a resist underlayer film material comprising a polymer prepared by copolymerizing a repeat unit of an acenaphthylene and a repeat unit having a substituted or unsubstituted hydroxy group has been suggested (see, for example, Patent Literature 3).

Meanwhile, as materials having high etching resistance for this kind of resist underlayer film, amorphous carbon underlayer films formed by CVD using methane gas, ethane gas, acetylene gas, or the like as a raw material are well known.

The present inventors have proposed an underlayer film forming composition for lithography containing a naphthalene formaldehyde polymer comprising a specific constituent unit, and an organic solvent (see, for example, Patent Literature 4 and Patent Literature 5) as a material that is excellent in optical properties and etching resistance and is also soluble in a solvent and applicable to a wet process.

As for methods for forming an intermediate layer used in the formation of a resist underlayer film in a three-layer process, for example, a method for forming a silicon nitride film (see, for example, Patent Literature 6) and a CVD formation method for a silicon nitride film (see, for example, Patent Literature 7) are known. Also, as intermediate layer materials for a three-layer process, materials comprising a silsesquioxane-based silicon compound are known (see, for example, Patent Literature 8 and Patent Literature 9).

### Citation List

### Patent Literature

Patent Literature 1: Japanese Patent Application Laid-Open No. 2004-177668
Patent Literature 2: Japanese Patent Application Laid-Open No. 2004-271838
Patent Literature 3: Japanese Patent Application Laid-Open No. 2005-250434
Patent Literature 4: International Publication No. WO 2009/072465
Patent Literature 5: International Publication No. WO 2011/034062

Patent Literature 6: Japanese Patent Application Laid-Open No. 2002-334869
Patent Literature 7: International Publication No. WO 2004/066377
Patent Literature 8: Japanese Patent Application Laid-Open No. 2007-226170
Patent Literature 9: Japanese Patent Application Laid-Open No. 2007-226204

Summary of Invention

Technical Problem

As mentioned above, a large number of film forming materials have heretofore been suggested. However, none of these materials not only have high solvent solubility that permits application of a wet process such as spin coating or screen printing but achieve all the heat resistance, etching resistance, embedding properties to a stepped substrate, and flatness of film to a higher level. Also, a large number of compositions intended for optical members have heretofore been suggested. However, none of these compositions achieve all of heat resistance, transparency and an index of refraction at a higher level. Thus, the development of novel materials is required.

The present invention has been made in view of the above-mentioned problems, and an object thereof is to provide a film forming material applicable to a wet process, a film for lithography excellent in the heat resistance, resist pattern shape, etching resistance, embedding properties to a stepped substrate, and flatness of film, and a film forming material useful for forming an optical component excellent in heat resistance, transparency, and refractive index. Another object of the present invention is to provide a composition for film formation for lithography, a material for optical component formation, a resist composition, a permanent film for a resist, a radiation-sensitive composition, an underlayer film forming material for lithography, and a composition for underlayer film formation for lithography, comprising the film forming material, and further to provide a resist pattern formation method, a method for producing an amorphous film, a method for producing an underlayer film for lithography, and a circuit pattern formation method using these.

Solution to Problem

The inventors have, as a result of devoted examinations to solve the problems, found out that use of a compound having a specific structure can solve the problems, and reached the present invention. More specifically, the present invention is as follows.

[1] A film forming material comprising a triazine-based compound represented by the following formula (1):

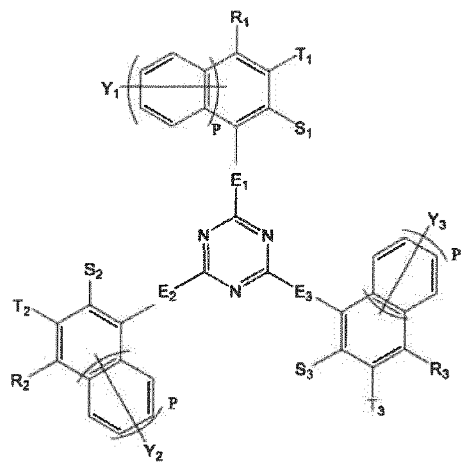

(1)

wherein $R_1$, $R_2$, and $R_3$ each independently represent a hydrogen atom, a linear alkyl group having 1 to 30 carbon atoms and optionally having a substituent, a branched alkyl group having 1 to 30 carbon atoms and optionally having a substituent, a cycloalkyl group having 3 to 30 carbon atoms and optionally having a substituent, an aryl group having 6 to 30 carbon atoms and optionally having a substituent, an alkenyl group having 2 to 30 carbon atoms and optionally having a substituent, an alkoxy group having 1 to 30 carbon atoms and optionally having a substituent, an alkylaryl group having 7 to 30 carbon atoms and optionally having a substituent, an arylalkyl group having 7 to 30 carbon atoms and optionally having a substituent, a hydroxyl group, or a group in which a hydrogen atom of a

hydroxyl group is replaced with an acid dissociation group or a crosslinkable reactive group, wherein the alkyl group, the cycloalkyl group, the aryl group, the alkenyl group, the alkoxy group, the alkylaryl group, or the arylalkyl group each optionally comprises an ether bond, a ketone bond, an ester bond, or a crosslinkable reactive group; $S_1$, $S_2$, and $S_3$ each independently represent a hydrogen atom, a hydroxyl group, a group in which a hydrogen atom of a hydroxyl group is replaced with an acid dissociation group or a crosslinkable reactive group, or an alkoxy group having 1 to 30 carbon atoms; $T_1$, $T_2$, and $T_3$ each independently represent a hydrogen atom, a hydroxyl group, an alkyl group having 1 to 30 carbon atoms, or an alkenyl group having 2 to 30 carbon atoms; $Y_1$, $Y_2$, and $Y_3$ each independently represent a hydrogen atom, a hydroxyl group, a group in which a hydrogen atom of a hydroxyl group is replaced with an acid dissociation group or a crosslinkable reactive group, an alkyl, alkoxy, alkoxycarbonyl or arylalkyl group having 1 to 30 carbon atoms, or an alkenyl group having 2 to 30 carbon atoms; $E_1$, $E_2$, and $E_3$ each independently represent a single bond, -O-, $-CH_2O-$, -COO-, or -NH-; and each P independently represents an integer of 0 to 1.

[2] The film forming material according to [1], wherein the triazine-based compound represented by the formula (1) is a triazine-based compound represented by the following formula (2):

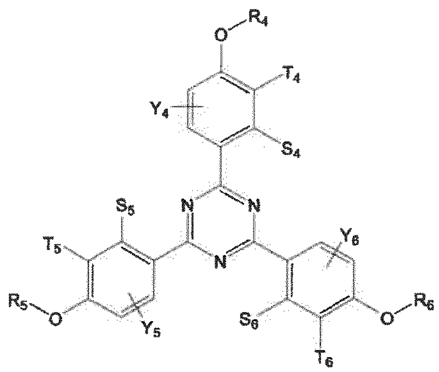

(2)

wherein $R_4$, $R_5$, and $R_6$ each independently represent a linear or branched alkyl group having 1 to 12 carbon atoms, a cycloalkyl group having 3 to 8 carbon atoms, an alkenyl group having 3 to 8 carbon atoms, an aryl group having 6 to 18 carbon atoms, or an alkylaryl or arylalkyl group having 7 to 18 carbon atoms, wherein the alkyl group, the cycloalkyl group, the alkenyl group, the aryl group, the alkylaryl group or the arylalkyl group is each optionally substituted with a hydroxyl group, or an alkyl or alkoxy group having 1 to 12 carbon atoms, and wherein the alkyl group, the cycloalkyl group, the alkenyl group, the aryl group, the alkylaryl group, or the arylalkyl group each optionally comprises an ether bond, a ketone bond, or an ester bond; $S_4$, $S_5$, and $S_6$ each independently represent a hydrogen atom, a hydroxyl group, a group in which a hydrogen atom of a hydroxyl group is replaced with an acid dissociation group or a crosslinkable reactive group, or an alkoxy group having 1 to 4 carbon atoms; $T_4$, $T_5$, and $T_6$ each independently represent a hydrogen atom, a hydroxyl group, an alkyl group having 1 to 8 carbon atoms, or an alkenyl group having 2 to 8 carbon atoms; and $Y_4$, $Y_5$, and $Y_6$ each independently represent a hydrogen atom, a hydroxyl group, a group in which a hydrogen atom of a hydroxyl group is replaced with an acid dissociation group or a crosslinkable reactive group, an alkyl, alkoxy, alkoxycarbonyl or arylalkyl group having 1 to 12 carbon atoms, or an alkenyl group having 2 to 8 carbon atoms.

[3] The film forming material according to [2], wherein the triazine-based compound represented by the formula (2) is a triazine-based compound represented by the following formula (3):

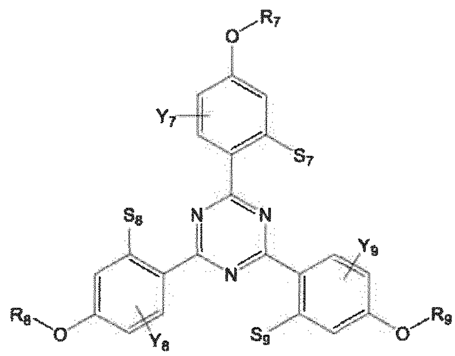

(3)

wherein $R_7$, $R_8$, and $R_9$ each independently represent a linear or branched alkyl group having 1 to 12 carbon atoms, a cycloalkyl group having 3 to 8 carbon atoms, or an alkenyl group having 3 to 8 carbon atoms, an aryl group having 6 to 18 carbon atoms, or an alkylaryl or arylalkyl group having 7 to 18 carbon atoms, wherein the alkyl group, the cycloalkyl group, the alkenyl group, the aryl group, the alkylaryl group or the arylalkyl group is each optionally substituted with a hydroxyl group, or an alkyl or alkoxy group having 1 to 12 carbon atoms, and wherein the alkyl group, the cycloalkyl group, the alkenyl group, the aryl group, the alkylaryl group, or the arylalkyl group each optionally comprises an ether bond, a ketone bond, or an ester bond; $S_7$, $S_8$, and $S_9$ each independently represent a hydrogen atom, a hydroxyl group, a group in which a hydrogen atom of a hydroxyl group is replaced with an acid dissociation group or a crosslinkable reactive group, or an alkoxy group having 1 to 4 carbon atoms; and $Y_7$, $Y_8$, and $Y_9$ each independently represent a hydrogen atom, a hydroxyl group, a group in which a hydrogen atom of a hydroxyl group is replaced with an acid dissociation group or a crosslinkable reactive group, an alkyl, alkoxy, alkoxycarbonyl or arylalkyl group having 1 to 12 carbon atoms, or an alkenyl group having 2 to 8 carbon atoms.

[4] The film forming material according to [3], wherein the triazine-based compound represented by the formula (3) is a triazine-based compound represented by the following formula (4):

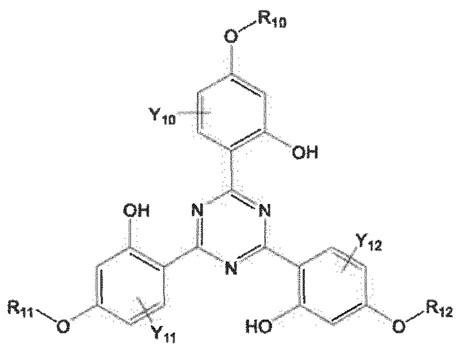

(4)

wherein $R_{10}$, $R_{11}$, and $R_{12}$ each independently represent a linear or branched alkyl group having 1 to 12 carbon atoms, a cycloalkyl group having 3 to 8 carbon atoms, or an alkenyl group having 3 to 8 carbon atoms, an aryl group having 6 to 18 carbon atoms, or an alkylaryl or arylalkyl group having 7 to 18 carbon atoms, wherein the alkyl group, the cycloalkyl group, the alkenyl group, the aryl group, the alkylaryl group or the arylalkyl group is each optionally substituted with a hydroxyl group, or an alkyl or alkoxy group having 1 to 12 carbon atoms, and wherein the alkyl group, the cycloalkyl group, the alkenyl group, the aryl group, the alkylaryl group, or the arylalkyl group each optionally comprises an ether bond, a ketone bond, or an ester bond; and $Y_{10}$, $Y_{11}$, and $Y_{12}$ each independently represent a hydrogen atom, a hydroxyl group, a group in which a hydrogen atom of a hydroxyl group is replaced with an acid dissociation group or a crosslinkable reactive group, an alkyl, alkoxy group, alkoxycarbonyl or arylalkyl group having 1 to 12 carbon atoms, or an alkenyl group having 2 to 8 carbon atoms.

[5] The film forming material according to [2], wherein the triazine-based compound represented by the formula (2) is a triazine-based compound represented by the following formula (5):

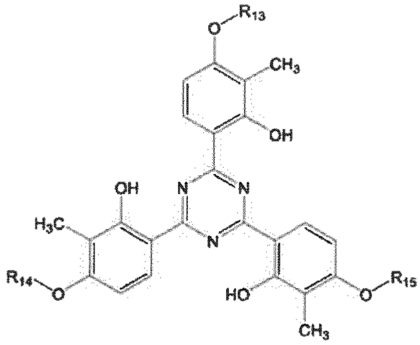

(5)

wherein $R_{13}$, $R_{14}$, and $R_{15}$ each independently represent a linear or branched alkyl group having 1 to 12 carbon atoms, wherein the alkyl group is optionally substituted with a hydroxyl group or an alkoxy group having 1 to 12 carbon atoms, and wherein the alkyl group optionally comprises an ether bond, a ketone bond or an ester bond.
[6] The film forming material according to [5], wherein the triazine-based compound represented by the formula (5) is a triazine-based compound represented by the following formula (BisN-8):

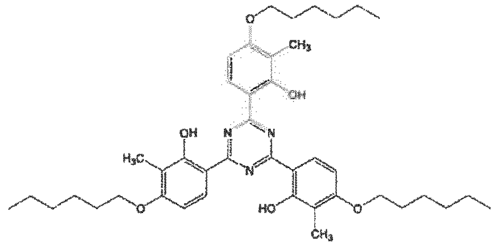

(BisN-8)

[7] A film forming material comprising a triazine-based compound represented by the following formula (6):

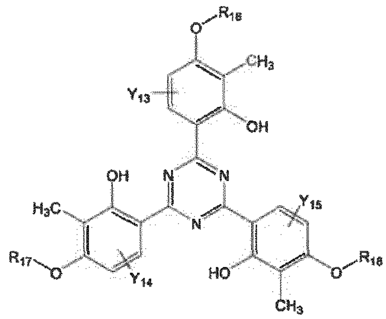

(6)

wherein $R_{16}$, $R_{17}$, and $R_{18}$ each independently represent a linear or branched alkyl group having 1 to 12 carbon atoms and substituted with a methacryloyloxy group or an acryloyloxy group, wherein the alkyl group is optionally substituted with a hydroxyl group, an alkoxy group having 1 to 8 carbon atoms, or an acyloxy group having 1 to 8 carbon atoms, and wherein the alkyl group optionally comprises an ether bond, a ketone bond or an ester bond; and $Y_{13}$, $Y_{14}$, and $Y_{15}$ each independently represent a hydrogen atom, a hydroxyl group, a group in which a hydrogen atom of a hydroxyl group is replaced with an acid dissociation group or a crosslinkable reactive group, an alkyl, alkoxy, alkoxycarbonyl or arylalkyl group having 1 to 12 carbon atoms, or an alkenyl group having 2 to 8 carbon atoms.
[8] The film forming material according to [7] further comprising:

one or more selected from the group consisting of a photocurable monomer, a photocurable oligomer, and a photocurable polymer, and
a photopolymerization initiator.

[9] A composition for film formation for lithography comprising one or more selected from the group consisting of

the film forming material according to any one of [1] to [8].
[10] A material for optical component formation comprising one or more selected from the group consisting of the film forming material according to any one of [1] to [8].
[11] A resist composition comprising one or more selected from the group consisting of the film forming material according to any one of [1] to [8].
[12] The resist composition according to [11], further comprising a solvent.
[13] The resist composition according to [11] or [12], further comprising an acid generating agent.
[14] The resist composition according to any one of [11] to [13], further comprising an acid diffusion controlling agent.
[15] A method for forming a resist pattern, comprising:

forming a resist film on a substrate using the resist composition according to any one of [11] to [14];
exposing at least a portion of the resist film; and
developing the exposed resist film, thereby forming a resist pattern.

[16] A permanent film for a resist obtained from the resist composition according to any one of [11] to [14].
[17] A radiation-sensitive composition comprising:

a component (A) which is one or more selected from the group consisting of the film forming material according to any one of [1] to [8],
an optically active diazonaphthoquinone compound (B), and
a solvent,
wherein a content of the solvent is 20 to 99% by mass based on 100% by mass in total of the radiation-sensitive composition.

[18] The radiation-sensitive composition according to [17], wherein a content ratio among the component (A), the optically active diazonaphthoquinone compound (B), and a further optional component (D) ((A)/(B)/(D)) is 1 to 99% by mass/99 to 1% by mass/0 to 98% by mass based on 100% by mass of solid components of the radiation-sensitive composition.
[19] The radiation-sensitive composition according to [17] or [18], wherein the radiation-sensitive composition is capable of forming an amorphous film by spin coating.
[20] A method for producing an amorphous film, comprising forming an amorphous film on a substrate using the radiation-sensitive composition according to any one of [17] to [19].
[21] A method for forming a resist pattern, comprising:

forming a resist film on a substrate using the radiation-sensitive composition according to any one of [17] to [19] ;
exposing at least a portion of the resist film; and
developing the exposed resist film, thereby forming a resist pattern.

[22] An underlayer film forming material for lithography comprising one or more selected from the group consisting of the film forming materials according to any one of [1] to [8].
[23] A composition for underlayer film formation for lithography comprising the underlayer film forming material for lithography according to [22], and a solvent.
[24] The composition for underlayer film formation for lithography according to [23], further comprising an acid generating agent.
[25] The composition for underlayer film formation for lithography according to [23] or [24], further comprising a crosslinking agent.
[26] A method for producing an underlayer film for lithography, comprising forming an underlayer film on a substrate using the composition for underlayer film formation for lithography according to any one of [23] to [25] .
[27] A method for forming a resist pattern, comprising:

forming an underlayer film on a substrate using the composition for underlayer film formation for lithography according to any one of [23] to [25] ;
forming at least one photoresist layer on the underlayer film; and
irradiating a predetermined region of the photoresist layer with radiation for development, thereby forming a resist pattern.

[28] A method for forming a circuit pattern, comprising:

forming an underlayer film on a substrate using the composition for underlayer film formation for lithography according to any one of [23] to [25] ;
forming an intermediate layer film on the underlayer film using a resist intermediate layer film material having a silicon atom;
forming at least one photoresist layer on the intermediate layer film;
irradiating a predetermined region of the photoresist layer with radiation for development, thereby forming a resist pattern;
etching the intermediate layer film with the resist pattern as a mask, thereby forming an intermediate layer film pattern;
etching the underlayer film with the intermediate layer film pattern as an etching mask, thereby forming an underlayer film pattern; and
etching the substrate with the underlayer film pattern as an etching mask, thereby forming a pattern on the substrate.

Advantageous Effects of Invention

According to the present invention, it is possible to provide a film forming material or the like, the film forming material being applicable to a wet process, being suitable for forming a film for lithography excellent in the heat resistance, resist pattern shape, etching resistance, embedding properties to a stepped substrate, and film flatness, and being suitable for forming a film forming material useful for forming an optical component excellent in heat resistance, transparency, and refractive index.

Description of Embodiments

Hereinafter, embodiments of the present invention (hereinafter, also simply referred to as the "present embodiment") will be described. The present embodiment is given in order to illustrate the present invention. The present invention is not limited to only the present embodiment.

[Film forming material]

The film forming material according to one aspect of the present embodiment comprises a triazine-based compound represented by the following formula (1). In the present specification, the "film" means, for example, those applicable to films for lithography, optical components, or the like (of course, not limited thereto), and typically is in a common form as a film for lithography or an optical component. That is, the "film forming material" is a precursor of such a film, and its form and/or composition is clearly distinguished from the "film". Further, the "film for lithography" is a concept broadly including, for example, a film for lithography applications such as a permanent film for a resist and an underlayer film for lithography.

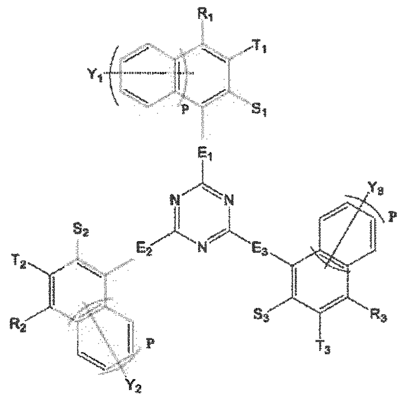

(1)

In the formula (1), $R_1$, $R_2$, and $R_3$ each independently represent a hydrogen atom, a linear alkyl group having 1 to 30 carbon atoms and optionally having a substituent, a branched alkyl group having 1 to 30 carbon atoms and optionally having a substituent, a cycloalkyl group having 3 to 30 carbon atoms and optionally having a substituent, an aryl group having 6 to 30 carbon atoms and optionally having a substituent, an alkenyl group having 2 to 30 carbon atoms and optionally having a substituent, an alkoxy group having 1 to 30 carbon atoms and optionally having a substituent, an alkylaryl group having 7 to 30 carbon atoms and optionally having a substituent, an arylalkyl group having 7 to 30 carbon atoms and optionally having a substituent, a hydroxyl group, or a group in which a hydrogen atom of a hydroxyl group is replaced with an acid dissociation group or a crosslinkable reactive group, wherein the alkyl group, the cycloalkyl group, the aryl group, the alkenyl group, the alkoxy group, the alkylaryl group, or the arylalkyl group each optionally contains an ether bond, a ketone bond, an ester bond, or a crosslinkable reactive group. $S_1$, $S_2$, and $S_3$ each independently represent a hydrogen atom, a hydroxyl group, or an alkoxy group having 1 to 30 carbon atoms, $T_1$, $T_2$, and $T_3$ each independently represent a hydrogen atom, a hydroxyl group, an alkyl group having 1 to 30 carbon atoms, or an alkenyl group having 2 to 30 carbon atoms, $Y_1$, $Y_2$, and $Y_3$ each independently represent a hydrogen atom, a hydroxyl group, a group in which a hydrogen atom of a hydroxyl group is replaced with an acid dissociation group or a crosslinkable reactive group, an alkyl, alkoxy group, alkoxycarbonyl or arylalkyl group having 1 to 30 carbon atoms, or an alkenyl group having 2 to 30 carbon atoms. $E_1$, $E_2$, and $E_3$ each independently represent a single bond, -O-, $-CH_2O-$, -COO-, or -NH-. Each P independently represents an integer of 0 to 1.

Herein, examples of the linear alkyl group having 1 to 30 carbon atoms and optionally having a substituent include, but not limited to, a methyl group, an ethyl group, a propyl group, a butyl group, a pentyl group, a hexyl group, a heptyl group, an octyl group, a nonyl group, a decyl group, a cyclopropylmethyl group, a cyclohexylmethyl group, and a 2-hydroxypropyl group, and a 2-methoxyethyl groups.

Examples of the branched alkyl group having 1 to 30 carbon atoms and optionally having a substituent include, but not limited to, a 1-methylethyl group, a 2-methylpropyl group, a 2-methylbutyl group, a 2-methylpentyl group, a 2-methylhexyl group, a 2-methylheptyl group, a 2-methyloctyl group, a 2-methylnonyl group, and a 2-methyldecyl group.

Examples of the cycloalkyl group having 3 to 30 carbon atoms and optionally having a substituent include, but not limited to, a cyclopropyl group, a cyclobutyl group, a cyclopentyl group, a cyclohexyl group, a cycloheptyl group, and a cyclooctyl group.

Examples of the aryl group having 6 to 30 carbon atoms and optionally having a substituent include, but not limited to, a phenyl group, a methylphenyl group, an ethylphenyl group, a propylphenyl group, a butylphenyl group, a pentylphenyl group, a hexylphenyl group, a heptylphenyl group, an octylphenyl group, a nonylphenyl group, a decylphenyl group, a dimethylphenyl group, a diethylphenyl group, a dipropylphenyl group, a dibutylphenyl group, a dipentylphenyl group, a dihexylphenyl group, a 1-methyl-1-phenylethyl group, a trimethylphenyl group, a triethylphenyl group, a butylmethylphenyl group, a butylethylphenyl group, a hydroxyphenyl group, a dihydroxyphenyl group, a tetrahydroxyphenyl group, a fluoromethylphenyl group, a fluoroethylphenyl group, a cyclohexylphenyl group, a methylcyclohexylphenyl group, an ethylcyclohexylphenyl group, a propylcyclohexylphenyl group, and a pentylcyclohexylphenyl group.

Examples of the alkenyl group having 2 to 30 carbon atoms and optionally having a substituent include, but not limited to, a propenyl group, a butenyl group, a pentenyl group, a hexenyl group, a heptenyl group, and an octenyl group.

Examples of the alkoxy group having 1 to 30 carbon atoms and optionally having a substituent include, but not limited to, a methoxy group, an ethoxy group, a propoxy group, an isopropoxy group, a butoxy group, a sec-butoxy group, a tert-butoxy group, a pentyloxy group, a hexyloxy group, a heptyloxy group, an octoxy group, and a decyloxy group.

Examples of the alkylaryl group having 7 to 30 carbon atoms include, but not limited to, a methylphenyl group, a dimethylphenyl group, an ethylphenyl group, and an octylphenyl group.

Examples of the arylalkyl group having 7 to 30 carbon atoms include, but not limited to, a benzyl group, a 2-phenylethyl group, and a 1-methyl-1-phenylethyl group.

In the present specification, the "acid dissociation group" refers to a characteristic group that is cleaved in the presence of an acid to cause a change to an alkali soluble group or the like. Examples of the alkali soluble group include, but not limited to, a phenolic hydroxy group, a carboxyl group, a sulfonic acid group, and a hexafluoroisopropanol group. A phenolic hydroxy group and a carboxyl group are preferable, and a phenolic hydroxy group is particularly preferable. The acid dissociation group can be appropriately selected and used from among those proposed in hydroxystyrene-based resins, (meth)acrylic acid-based resins, and the like for use in chemically amplified resist compositions for KrF or ArF and is not particularly limited. Examples of the acid dissociation group include, but not limited to, the acid dissociation groups described in JP 2012-136520A.

In the present specification, the "crosslinkable reactive group" refers to a group that crosslinks in the presence or absence of a catalyst. Examples of the crosslinkable reactive group include, but not particularly limited to, a group having an alkoxy group having 1 to 20 carbon atoms and an allyl group, a group having a (meth)acryloyl group, a group having an epoxy (meth)acryloyl group, and a group having a hydroxyl group, a group having a urethane (meth) acryloyl group, a group having a glycidyl group, a group having a vinyl-containing phenylmethyl group, and a group having a styrene group.

Examples of the group having an allyl group include, but not particularly limited to, a group represented by the following formula (X-1).

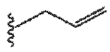 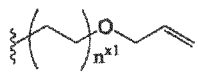 (X-1)

In the formula (X-1), $n^{X1}$ is an integer of 1 to 5.

Examples of the group having a (meth)acryloyl group include, but not particularly limited to, a group represented by the following formula (X-2).

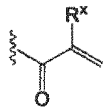 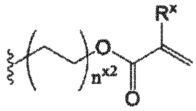 (X-2)

In the formula (X-2), $n^{X2}$ is an integer of 1 to 5, and $R^X$ is a hydrogen atom or a methyl group.

Examples of the group having an epoxy (meth)acryloyl group include, but not particularly limited to, a group represented by the following formula (X-3). Here, the epoxy (meth)acryloyl group refers to a group generated by reacting epoxy (meth)acrylate and a hydroxyl group.

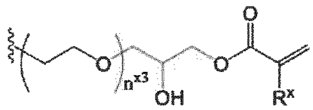 (X-3)

In the formula (X-3), $n^{X3}$ is an integer of 0 to 5, and $R^X$ is a hydrogen atom or a methyl group.

Examples of the group having a urethane (meth)acryloyl group include, but not particularly limited to, a group represented by the following formula (X-4).

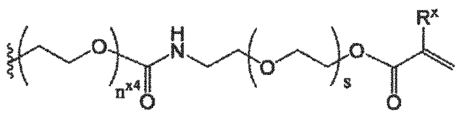 (X-4)

In the formula (X-4), $n^{X4}$ is an integer of 0 to 5, s is an integer of 0 to 3, and $R^X$ is a hydrogen atom or a methyl group.

Examples of the group having a hydroxyl group include, but not particularly limited to, a group represented by the following formula (X-5).

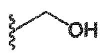 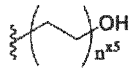 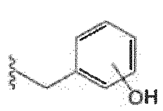 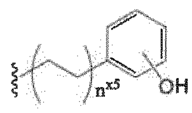 (X-5)

In the formula (X-5), $n^{X5}$ is an integer of 1 to 5.

Examples of the group having a glycidyl group include, but not particularly limited to, a group represented by the following formula (X-6).

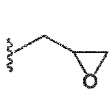 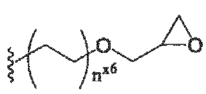 (X-6)

In the formula (X-6), $n^{X6}$ is an integer of 1 to 5.

Examples of the group having a vinyl-containing phenylmethyl group include, but not particularly limited to, a group represented by the following formula (X-7).

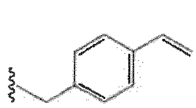 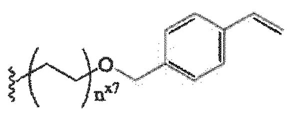 (X-7)

In the formula (X-7), $n^{X7}$ is an integer of 1 to 5.

Examples of the group having a styrene group include, but not particularly limited to, a group represented by the following formula (X-8).

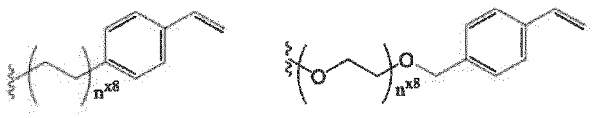

(X－8)

In the formula (X-8), $n^{X8}$ is an integer of 1 to 5.

Among the above, from the viewpoint of ultraviolet curability, the crosslinkable reactive group is preferably a group having a (meth)acryloyl group, an epoxy (meth)acryloyl group, a urethane (meth)acryloyl group, or a glycidyl group, or a group containing a styrene group, more preferably a group having a (meth)acryloyl group, an epoxy (meth)acryloyl group, or a urethane (meth)acryloyl group, and even more preferably a group having a (meth)acryloyl group.

$R_1$, $R_2$, and $R_3$ is preferably a hydrogen atom, a hydroxyl group, a methyl group, an ethyl group, a propyl group, a butyl group, a 1-methylethyl group, a 2-methylpropyl group, a 2-methylbutyl group, a 2-methylpentyl group, a cyclopropyl group, a cyclobutyl group, a cyclopentyl group, a cyclohexyl group, a methylphenyl group, an ethylphenyl group, a propylphenyl group, or a butylphenyl group,a propenyl group, a butenyl group, a methoxy group, an ethoxy group, a propoxy group, an isopropoxy group, a butoxy group, a sec-butoxy group, a tert-butoxy group, a pentyloxy group and a hexyloxy group from the viewpoint of solvent solubility and heat resistance.

$S_1$, $S_2$, and $S_3$ in the formula (1) each independently is a hydrogen atom, a hydroxyl group, or an alkoxy group having 1 to 30 carbon atoms. Examples of the alkoxy group having 1 to 30 carbon atoms include, but not limited to, a methoxy group, an ethoxy group, a propoxy group, an isopropoxy group, and a butoxy group.

$S_1$, $S_2$, and $S_3$ is preferably a hydrogen atom, a hydroxyl group, or a methyl group, and more preferably a hydroxyl group from the viewpoint of solubility and heat resistance.

$T_1$, $T_2$, and $T_3$ in the formula (1) each independently is a hydrogen atom, a hydroxyl group, an alkyl group having 1 to 30 carbon atoms, or an alkenyl group having 2 to 30 carbon atoms.

Examples of the alkyl group having 1 to 30 carbon atoms include, but not limited to, a methyl group, an ethyl group, a propyl group, an isopropyl group, a butyl group, a sec-butyl group, a tert-butyl group, an isobutyl group, an amyl group, a tert-amyl group, an octyl group, and a tert-octyl group.

Examples of the alkenyl group having 2 to 30 carbon atoms include, but not limited to, a vinyl group, a propenyl group, a butenyl group, a pentenyl group, a hexenyl group, a heptenyl group, and an octenyl group.

$T_1$, $T_2$, and $T_3$ is preferably a hydrogen atom, a hydroxyl group, or a methyl group from the viewpoint of solubility and heat resistance.

$Y_1$, $Y_2$, and $Y_3$ in the formula (1) each independently is a hydrogen atom, a hydroxyl group, a group in which a hydrogen atom of a hydroxyl group is replaced with an acid dissociation group or a crosslinkable reactive group, an alkyl, alkoxy, alkoxycarbonyl or arylalkyl group having 1 to 30 carbon atoms, or an alkenyl group having 2 to 30 carbon atoms.

Examples of the alkyl group having 1 to 30 carbon atoms include a linear or branched alkyl group having 1 to 30 carbon atoms. Herein, examples of the linear alkyl group having 1 to 30 carbon atoms and optionally having a substituent include, but not limited to, a methyl group, an ethyl group, a propyl group, a butyl group, a pentyl group, a hexyl group, a heptyl group, an octyl group, a nonyl group, a decyl group, a cyclopropylmethyl group, a cyclohexylmethyl group, a 2-hydroxypropyl, and a 2-methoxyethyl group. Examples of the branched alkyl group having 1 to 30 carbon atoms include, but not limited to, a 1-methylethyl group, a 2-methylpropyl group, a 2-methylbutyl group, a 2-methylpentyl group, a 2-methylhexyl group, a 2-methylheptyl group, a 2-methyloctyl group, a 2-methylnonyl group, and a 2-methyldecyl group.

Examples of the alkoxy group having 1 to 30 carbon atoms include, but not limited to, a methoxy group, an ethoxy group, a propoxy group, an isopropoxy group, a butoxy group, a sec-butoxy group, a tert-butoxy group, a pentyloxy group, a hexyloxy group, a heptyloxy group, an octoxy group, and a decyloxy group.

Examples of the alkoxycarbonyl group having 1 to 30 carbon atoms include, but not limited to, derivatives of the above alkoxy groups.

Examples of the arylalkyl group having 1 to 30 carbon atoms include, but not limited to, a cumyl group, and a phenylmethylene group.

Examples of the alkenyl group having 2 to 30 carbon atoms include, but not limited to, a propenyl group, a butenyl group, a pentenyl group, a hexenyl group, a heptenyl group, and an octenyl group.

$Y_1$, $Y_2$, and $Y_3$ is preferably a hydrogen atom, a hydroxyl group, a group in which a hydrogen atom of a hydroxyl group is replaced with an acid dissociation group or a crosslinkable reactive group, or a methyl group from the viewpoint of solubility and heat resistance.

In the present embodiment, in a case where the film forming material is used to form a film for lithography, from the viewpoint of further improving applicability to a wet process, heat resistance, resist pattern shape, etching resistance, embedding properties to a stepped substrate and flatness of film, and in a case where the film forming material is used to form an optical component, from the viewpoint of further improving heat resistance, resist pattern shape processability, transparency, and refractive index, it is preferable that at least one of $R_1$ to $R_3$ in the formula (1) have an acid dissociation group and/or a crosslinkable reactive group. From the same viewpoint, it is preferable that at least one of $S_1$ to $S_3$ in the formula (1) have an acid dissociation group and/or a crosslinkable reactive group. Further, from the same viewpoint, it is preferable that at least one of $Y_1$ to $Y_3$ in the formula (1) have an acid dissociation group and/or a crosslinkable reactive group.

The content of the triazine-based compound represented by the formula (1) in the film forming material of the present embodiment is preferably 50 to 100% by mass, more preferably 60 to 100% by mass, even more preferably 70 to 100% by mass, and particularly preferably 80 to 100% by mass from the viewpoint of heat resistance and etching resistance.

The triazine-based compound in the film forming material of the present embodiment has the structure as described above and therefore has both high heat resistance and high solvent solubility. The triazine-based compound represented by the formula (1) of the present embodiment is also preferably a compound represented by the following formula (2) from the viewpoint of solubility in a solvent and heat resistance.

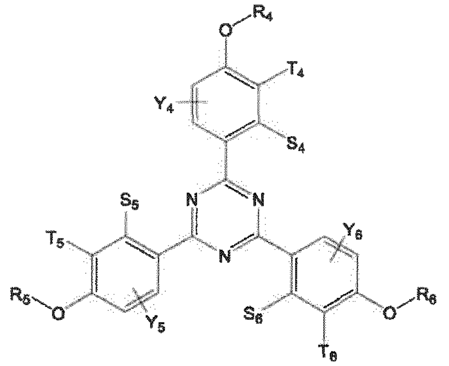

(2)

In the formula (2), $R_4$, $R_5$, and $R_6$ each independently represent a linear or branched alkyl group having 1 to 12 carbon atoms, a cycloalkyl group having 3 to 8 carbon atoms, or an alkenyl group having 3 to 8 carbon atoms, an aryl group having 6 to 18 carbon atoms, or an alkylaryl or arylalkyl group having 7 to 18 carbon atoms. Here, the alkyl group, the cycloalkyl group, the alkenyl group, the aryl group, the alkylaryl group or the arylalkyl group is each optionally substituted with a hydroxyl group, or an alkyl or alkoxy group having 1 to 12 carbon atoms. The alkyl group, the cycloalkyl group, the alkenyl group, the aryl group, the alkylaryl group, or the arylalkyl group each optionally contains an ether bond, a ketone bond, or an ester bond. $S_4$, $S_5$, and $S_6$ each independently represent a hydrogen atom, a hydroxyl group, a group in which a hydrogen atom of a hydroxyl group is replaced with an acid dissociation group or a crosslinkable reactive group, or an alkoxy group having 1 to 4 carbon atoms, $T_4$, $T_5$, and $T_6$ each independently represent a hydrogen atom, a hydroxyl group, an alkyl group having 1 to 8 carbon atoms, or an alkenyl group having 2 to 8 carbon atoms, and $Y_4$, $Y_5$, and $Y_6$ each independently represent a hydrogen atom, a hydroxyl group, a group in which a hydrogen atom of a hydroxyl group is replaced with an acid dissociation group or a crosslinkable reactive group, an alkyl, alkoxy, alkoxycarbonyl or arylalkyl group having 1 to 12 carbon atoms, or an alkenyl group having 2 to 8 carbon atoms.

Herein, examples of the linear alkyl group having 1 to 12 carbon atoms include, but not limited to, a methyl group, an ethyl group, a propyl group, a butyl group, a pentyl group, a hexyl group, a heptyl group, an octyl group, a nonyl group, a decyl group, an icosyl group, a triacontyl group, a cyclopropylmethyl group, a cyclohexylmethyl group and an adamantylmethyl group.

Examples of the branched alkyl group having 1 to 12 carbon atoms and optionally having a substituent include, but not limited to, a 1-methylethyl group, a 2-methylpropyl group, a 2-methylbutyl group, a 2-methylpentyl group, a 2-methylhexyl group, a 2-methylheptyl group, a 2-methyloctyl group, a 2-methylnonyl group, a 2-methyldecyl group, a 2-methylicosyl group, and a 2-methylnonacosyl group.

Examples of the cycloalkyl group having 3 to 8 carbon atoms include, but not limited to, a cyclopropyl group, a cyclobutyl group, a cyclopentyl group, a cyclohexyl group, a cycloheptyl group, a cyclooctyl group, a cyclononyl group, a cyclodecyl group, a cyclooctadecylene group, and an adamantyl group.

Examples of the aryl group having 6 to 18 carbon atoms include, but not limited to, a methylphenyl group, an ethylphenyl group, a propylphenyl group, a butylphenyl group, a pentylphenyl group, a hexylphenyl group, a heptylphenyl

group, an octylphenyl group, a nonylphenyl group, a decylphenyl group, an icosylphenyl group, a pentacosylphenyl group, a dimethylphenyl group, a diethylphenyl group, a dipropylphenyl group, a dibutylphenyl group, a dipentylphenyl group, a dihexylphenyl group, a diheptylphenyl group, a dioctylphenyl group, a dinonylphenyl group, a didecylphenyl group, a didodecylphenyl group, a trimethylphenyl group, a triethylphenyl group, a butylmethylphenyl group, a butylethylphenyl group, a hydroxyphenyl group, a dihydroxyphenyl group, a tetrahydroxyphenyl group, a fluoromethylphenyl group, a fluoroethylphenyl group, a cyclohexylphenyl group, a cyclohexylnaphthyl group, a methylcyclohexylphenyl group, an ethylcyclohexylphenyl group, a propylcyclohexylphenyl group, and a pentylcyclohexylphenyl group.

Examples of the alkenyl group having 3 to 8 carbon atoms include, but not limited to, a linear or branched propenyl group, a butenyl group, a pentenyl group, a hexenyl group, a heptenyl group, and an octenyl group.

Examples of the alkylaryl group having 7 to 18 carbon atoms include, but not limited to, a methylphenyl group, a dimethylphenyl group, an ethylphenyl group, and an octylphenyl group.

Examples of the arylakyl group having 7 to 18 carbon atoms include, but not limited to, a benzyl group, a 2-phenylethyl group, and a 1-methyl-1-phenylethyl group.

Examples of the alkoxy group having 1 to 12 carbon atoms include, but not limited to, a methoxy group, an ethoxy group, a propoxy group, a butoxy group, a pentoxy group, a hexaoxy group, an octoxy group, a nonyloxy group, a decyloxy group, an undecyloxy group, and a decyloxy group.

Among these, $R_4$, $R_5$, and $R_6$ is preferably a hydrogen atom, a methyl group, an ethyl group, a propyl group, a butyl group, a 1-methylethyl group, a 2-methylpropyl group, a 2-methylbutyl group, a 2-methylpentyl group, a cyclopropyl group, a cyclobutyl group, a cyclopentyl group, a cyclohexyl group, a methylphenyl group, an ethylphenyl group, a propylphenyl group, a butylphenyl group,a propenyl group, a butenyl group, a methoxy group, an ethoxy group, a propoxy group, an isopropoxy group, a butoxy group, a sec-butoxy group, a tert-butoxy group, a pentyloxy group, or a hexyloxy group from the viewpoint of solvent solubility and heat resistance.

Examples of $S_4$, $S_5$, and $S_6$ in the formula (2) include the groups exemplified as $S_1$, $S_2$ and $S_3$ in the formula (1).

Examples of $T_4$, $T_5$, and $T_6$ in the formula (2) include the groups exemplified as $T_1$, $T_2$, and $T_3$ in the formula (1) .

Examples of $Y_4$, $Y_5$, and $Y_6$ in the formula (2) include the groups exemplified as $Y_1$, $Y_2$, and $Y_3$ in the formula (1) .

The triazine-based compound represented by the formula (2) is further preferably a compound represented by the following formula (3) from the viewpoint of solubility in a solvent and heat resistance.

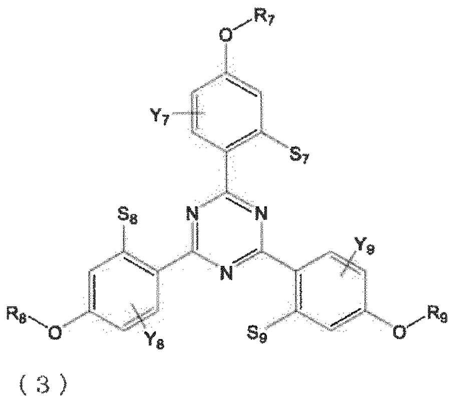

(3)

In the formula (3), $R_7$, $R_8$, and $R_9$ each independently represent a linear or branched alkyl group having 1 to 12 carbon atoms, a cycloalkyl group having 3 to 8 carbon atoms, or an alkenyl group having 3 to 8 carbon atoms, an aryl group having 6 to 18 carbon atoms, or an alkylaryl or arylalkyl group having 7 to 18 carbon atoms. Here, the alkyl group, the cycloalkyl group, the alkenyl group, the aryl group, the alkylaryl group or the arylalkyl group is each optionally substituted with a hydroxyl group, or an alkyl or alkoxy group having 1 to 12 carbon atoms. The alkyl group, the cycloalkyl group, the alkenyl group, the aryl group, the alkylaryl group, or the arylalkyl group each optionally contains an ether bond, a ketone bond, or an ester bond. $S_7$, $S_8$, and $S_9$ each independently represent a hydrogen atom, a hydroxyl group, a group in which a hydrogen atom of a hydroxyl group is replaced with an acid dissociation group or a crosslinkable reactive group, or an alkoxy group having 1 to 4 carbon atoms. $Y_7$, $Y_8$, and $Y_9$ each independently represent a hydrogen atom, a hydroxyl group, a group in which a hydrogen atom of a hydroxyl group is replaced with an acid dissociation group or a crosslinkable reactive group, an alkyl, alkoxy, alkoxycarbonyl or arylalkyl group having 1 to 12 carbon atoms, or an alkenyl group having 2 to 8 carbon atoms.

Examples of $R_7$, $R_8$, and $R_9$ in the formula (3) include the groups exemplified as $R_4$, $R_5$, and $R_6$ in the formula (2) .

Examples of $S_7$, $S_8$, and $S_9$ in the formula (3) include the groups exemplified as $S_4$, $S_5$, and $S_6$ in the formula (2) .

Examples of $Y_7$, $Y_8$, and $Y_9$ in the formula (3) include the groups exemplified as $Y_4$, $Y_5$, and $Y_6$ in the formula (2) .

The triazine-based compound represented by the formula (3) is further preferably a triazine-based compound

represented by the following formula (4) from the viewpoint of solubility in a solvent and heat resistance.

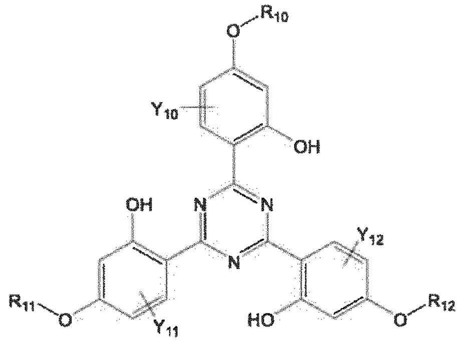

(4)

In the formula (4), $R_{10}$, $R_{11}$, and $R_{12}$ each independently represent a linear or branched alkyl group having 1 to 12 carbon atoms, a cycloalkyl group having 3 to 8 carbon atoms, or an alkenyl group having 3 to 8 carbon atoms, an aryl group having 6 to 18 carbon atoms, or an alkylaryl or arylalkyl group having 7 to 18 carbon atoms. Here, the alkyl group, the cycloalkyl group, the alkenyl group, the aryl group, the alkylaryl group or the arylalkyl group is each optionally substituted with a hydroxyl group, or an alkyl or alkoxy group having 1 to 12 carbon atoms. The alkyl group, the cycloalkyl group, the alkenyl group, the aryl group, the alkylaryl group, or the arylalkyl group each optionally contains an ether bond, a ketone bond, or an ester bond. $Y_{10}$, $Y_{11}$, and $Y_{12}$ each independently represent a hydrogen atom, a hydroxyl group, a group in which a hydrogen atom of a hydroxyl group is replaced with an acid dissociation group or a crosslinkable reactive group, an alkyl, alkoxy, alkoxycarbonyl or arylalkyl group having 1 to 12 carbon atoms, or an alkenyl group having 2 to 8 carbon atoms.

Examples of $R_{10}$, $R_{11}$, and $R_{12}$ in the formula (4) include the groups exemplified as $R_7$, $R_8$, and $R_9$ in the formula (3).

Examples of $Y_{10}$, $Y_{11}$, and $Y_{12}$ in the formula (4) include the groups exemplified as $Y_7$, $Y_8$, and $Y_9$ in the formula (3).

The triazine-based compound represented by the formula (2) is further preferably a triazine-based compound represented by the following formula (5) from the viewpoint of heat resistance.

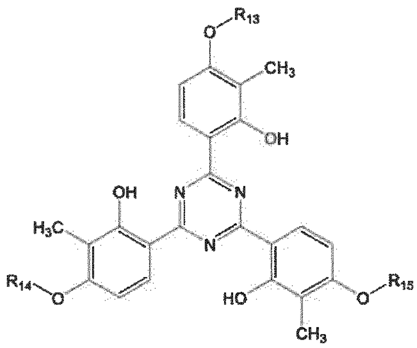

(5)

In the formula (5), $R_{13}$, $R_{14}$, and $R_{15}$ each independently represent a linear or branched alkyl group having 1 to 12 carbon atoms, wherein the alkyl group is optionally substituted with a hydroxyl group or an alkoxy group having 1 to 12 carbon atoms. The alkyl group optionally contains an ether bond, a ketone bond or an ester bond.

Examples of the linear or branched alkyl group having 1 to 12 carbon atoms and the alkoxy group having 1 to 12 carbon atoms as $R_{13}$, $R_{14}$, and $R_{15}$ in the formula (5) include the groups exemplified as $R_4$, $R_5$, and $R_6$ in the formula (2).

[Photocurable film forming material]

The film forming material according to another aspect of the present embodiment contains a triazine-based compound represented by the following formula (6), and may be preferably used particularly as a material for forming a photocurable film.

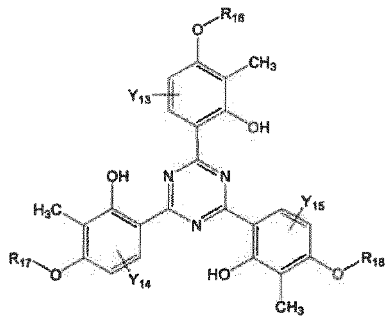

(6)

wherein $R_{16}$, $R_{17}$, and $R_{18}$ each independently represent a linear or branched alkyl group having 1 to 12 carbon atoms and substituted with a methacryloyloxy group or an acryloyloxy group, wherein the alkyl group is optionally substituted with a hydroxyl group, an alkoxy group having 1 to 8 carbon atoms, or an acyloxy group having 1 to 8 carbon atoms. The alkyl group optionally contains an ether bond, a ketone bond or an ester bond. $Y_{13}$, $Y_{14}$, and $Y_{15}$ each independently represent a hydrogen atom, a hydroxyl group, a group in which a hydrogen atom of a hydroxyl group is replaced with an acid dissociation group or a crosslinkable reactive group, an alkyl, alkoxy, alkoxycarbonyl or arylalkyl group having 1 to 12 carbon atoms, or an alkenyl group having 2 to 8 carbon atoms).

In the formula (6), the methacryloyloxy group is a group represented by the following formula (7) in which Z is a methyl group, and the acryloyloxy group is a group represented by the following formula (7) in which Z is a hydrogen atom.

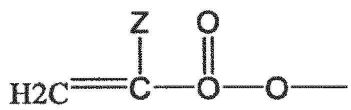

(7)

wherein Z represents a hydrogen atom or a methyl group.

Examples of the linear or branched alkyl group having 1 to 12 carbon atoms and the alkoxy group having 1 to 8 carbon atoms as $R_{16}$, $R_{17}$, and $R_{18}$ in the formula (6) include the groups exemplified as $R_4$, $R_5$, and $R_6$ in the formula (2).

Further, $R_{16}$, $R_{17}$, and $R_{18}$ are preferably alkyl groups having 1 to 8 carbon atoms, which are substituted with the acryloyloxy group, which is superior in ultraviolet absorbability and ultraviolet curability to the methacryloyloxy group.

The position of substitution by the methacryloyloxy group or the acryloyloxy group may be any position of the linear or branched alkyl group having 1 to 12 carbon atoms.

Examples the acyloxy group having 1 to 8 carbon atoms include an acyloxy group corresponding to the alkyl group having 1 to 8 carbon atoms among the exemplified linear or branched alkyl groups having 1 to 12 carbon atoms.

Examples of $Y_{13}$, $Y_{14}$, and $Y_{15}$ in the formula (6) include the groups exemplified as $Y_4$, $Y_5$, and $Y_6$ in the formula (2).

The film forming material of the present embodiment may further contain, in addition to the triazine-based compound represented by the formula (6), one or more selected from the group consisting of a photocurable monomer, a photocurable oligomer, a photocurable polymer, and a photopolymerization initiator.

The photocurable monomer, the photocurable oligomer, and the photocurable polymer are preferably a compound having one or more radically polymerizable functional groups, and preferably a (meth)acrylate compound. The content of the photocurable monomer, the photocurable oligomer, and the photocurable polymer is preferably 80 to 95% by mass with respect to the entire photocurable film forming material.

Examples of the photopolymerization initiator include, but not limited to, IRGACURE 651, IRGACURE 184, IRGACURE 907, IRGACURE 369E, IRGACURE 819, IRGACURE OXE01, and IRGACURE OXE02 manufactured by BASF Corporation.

The content of the photopolymerization initiator is preferably 0.1 to 10% by mass with respect to the entire photocurable film forming material.

Specific examples of the triazine-based compound according to the present embodiment include compounds such as the compounds BisN-1 to BisN-19 shown below, but are not limited to those listed here.

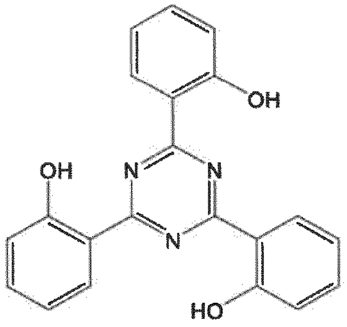

BisN－1

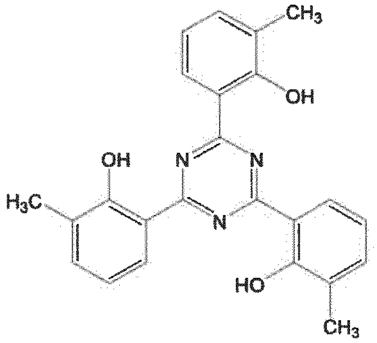

BisN－2

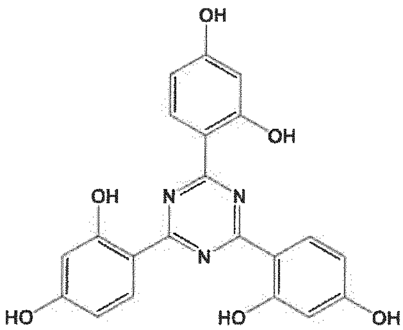

BisN－3

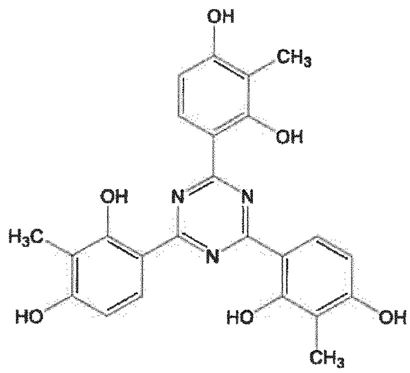

BisN－4

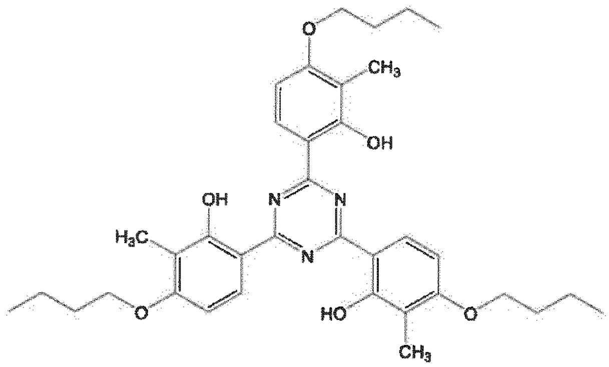

ＢｉｓＮ－５

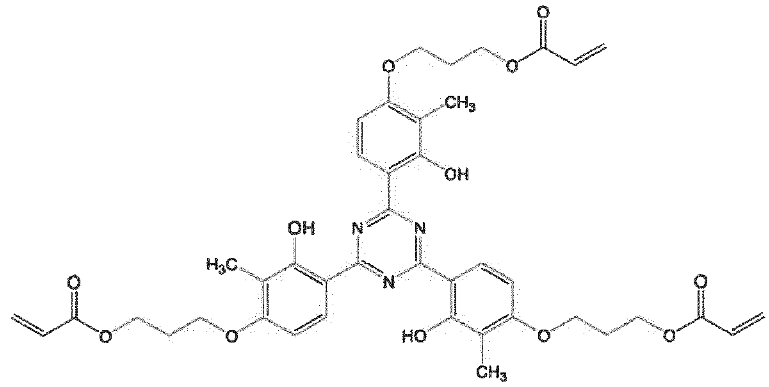

ＢｉｓＮ－６

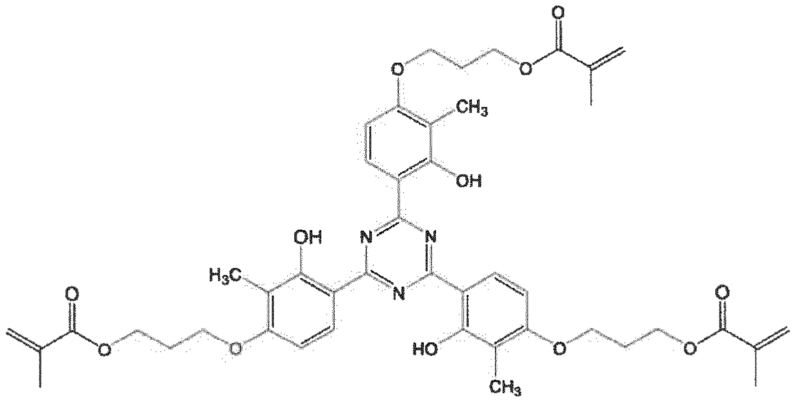

ＢｉｓＮ－７

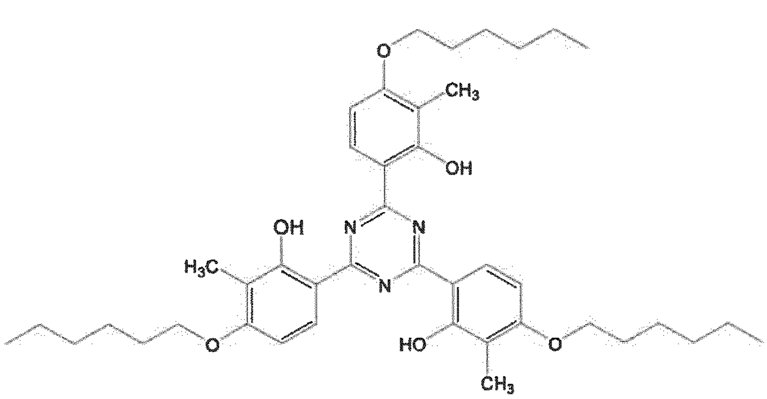

ＢｉｓＮ－８

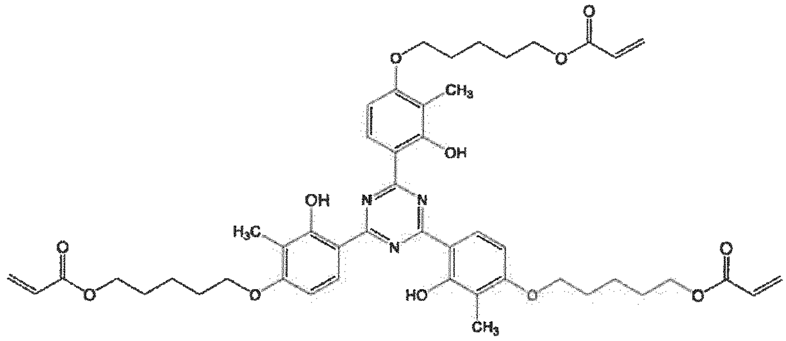

ＢｉｓＮ－９

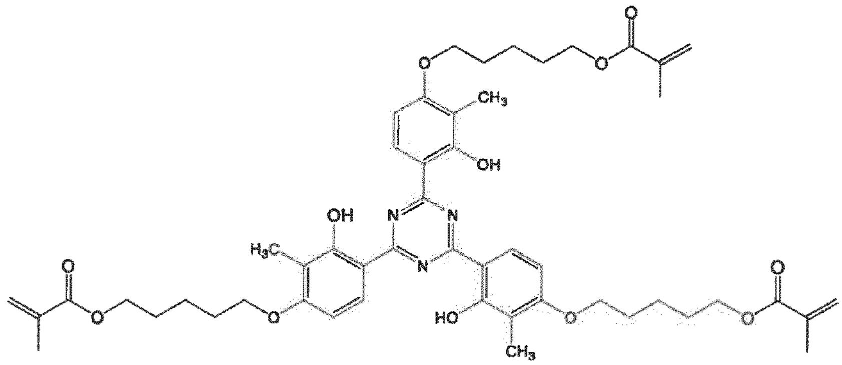

ＢｉｓＮ－１０

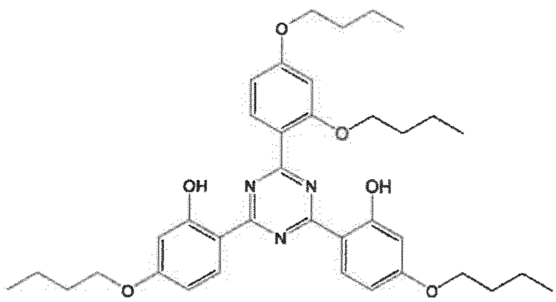

ＢｉｓＮ－１１

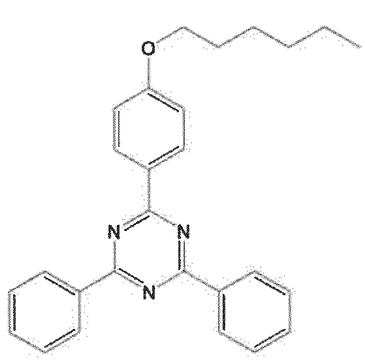

ＢｉｓＮ－１２

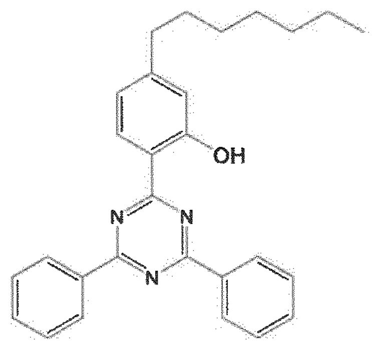

BisN-13

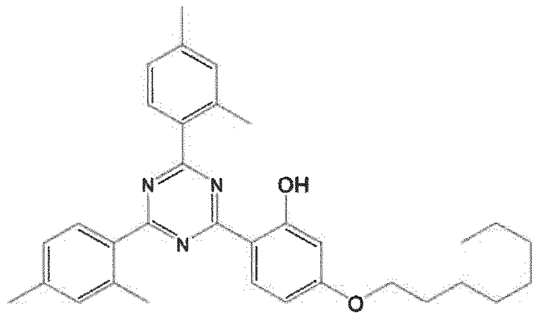

BisN-14

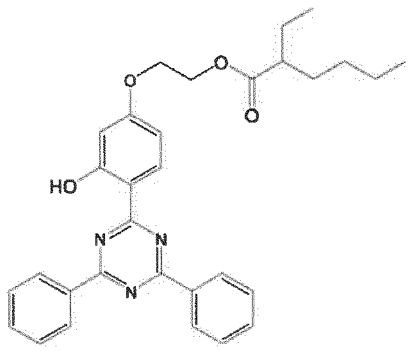

BisN-15

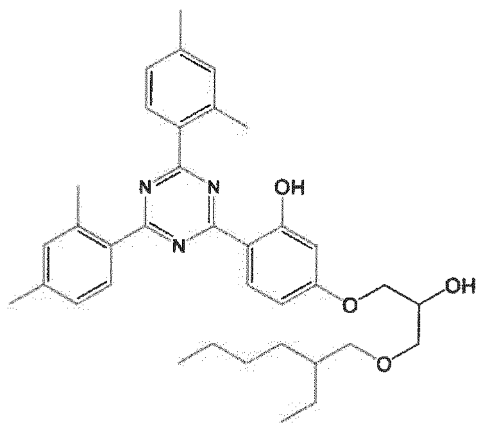

BisN-16

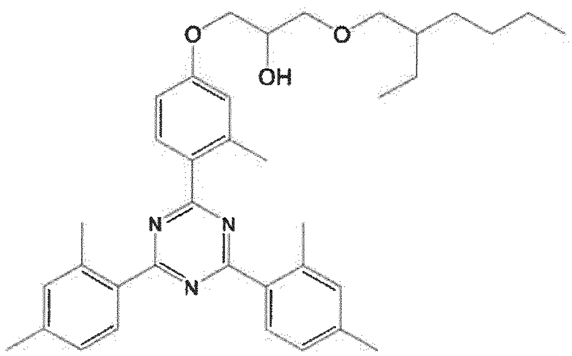

BisN-17

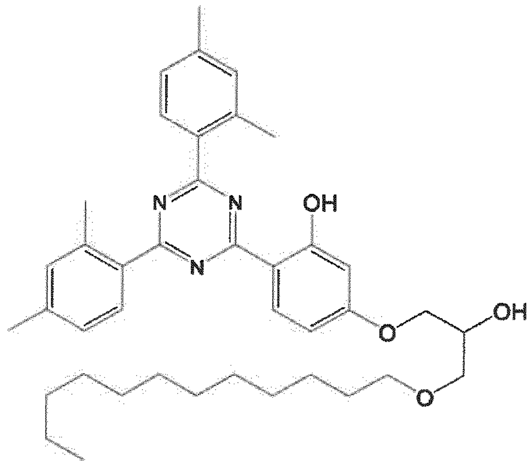

BisN-18

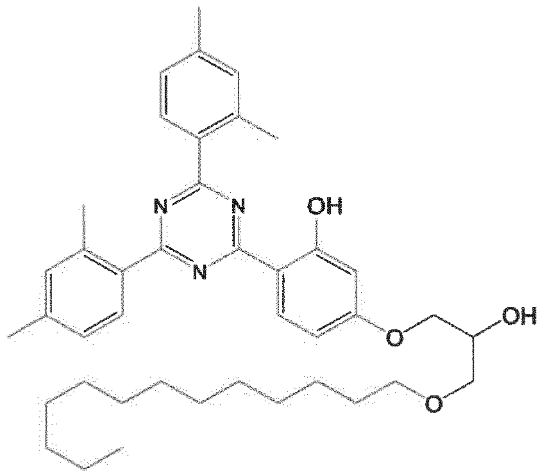

BisN-19

The compound represented by the formula (5) is preferably a triazine-based compound represented by the formula (BisN-8) from the viewpoint of heat resistance.

The film forming material of the present embodiment has a rigid triazine skeleton, is susceptible to a crosslinking reaction by high temperature baking, and develops high heat resistance. As a result, in the application for forming a film for lithography, it is possible to form an underlayer film for lithography that is prevented from deterioration of the film during high temperature baking and is excellent in etching resistance against oxygen plasma etching or the like. The film forming material of the present embodiment has high solubility in organic solvents and high solubility in safe solvents despite having an aromatic structure. The underlayer film for lithography including the composition for film formation for

lithography of the present embodiment described later is excellent in embedding properties to a stepped substrate and flatness of film, so that an excellent resist pattern can be obtained. Moreover, the film forming material of the present embodiment achieves both high refractive index and heat resistance at a high level by introducing a triazine ring.

A film forming composition for lithography according to the present embodiment contains the above film forming material of the present embodiment. As described above, the film forming material according to the present embodiment contains the compound represented by the formula (1) and one or more substances selected from the compound group, and hereinafter, "compound represented by the formula (1) and one or more selected from the compound group" is also referred to as "the compound of the present embodiment" or "component (A)".

[Material for optical component formation]

The material for optical component formation of the present embodiment contains the above film forming material according to the present embodiment. Herein, the "optical component" refers to a component in the form of a film or a sheet as well as a plastic lens (a prism lens, a lenticular lens, a microlens, a Fresnel lens, a viewing angle control lens, a contrast improving lens, etc.), a phase difference film, a film for electromagnetic wave shielding, a prism, an optical fiber, a solder resist for flexible printed wiring, a plating resist, an interlayer insulating film for multilayer printed circuit boards, or a photosensitive optical waveguide. The compounds according to the present embodiment are useful for forming these optical components.

[Resist composition]

The resist composition of the present embodiment contains the above film forming material according to the present embodiment.

It is preferable that the resist composition of the present embodiment further contains a solvent. The solvent is not particularly limited, and, for example, an acid generating agent described in International Publication No. WO2013/024778 can be used. These solvents can be used alone or in combination of two or more kinds.

The solvent is preferably a safe solvent, more preferably at least one selected from PGMEA (propylene glycol monomethyl ether acetate), PGME (propylene glycol monomethyl ether), CHN (cyclohexanone), CPN (cyclopentanone), orthoxylene (OX), 2-heptanone, anisole, butyl acetate, ethyl propionate, and ethyl lactate.

In the present embodiment, the amount of the solid component and the amount of the solvent are not particularly limited, but preferably the solid component is 1 to 80% by mass and the solvent is 20 to 99% by mass, more preferably the solid component is 1 to 50% by mass and the solvent is 50 to 99% by mass, even more preferably the solid component is 2 to 40% by mass and the solvent is 60 to 98% by mass, and particularly preferably the solid component is 2 to 10% by mass and the solvent is 90 to 98% by mass, based on 100% by mass of the total mass of the amount of the solid component and the solvent.

[Other components]

The resist composition of the present embodiment may optionally contain other components such as a crosslinking agent, an acid generating agent, and an organic solvent in addition to the compound having a structure represented by the formula (1) and one or more substances selected from the compound group. Hereinafter, these optional components will be described.

[Acid generating agent (C)]

The resist composition of the present embodiment preferably contains one or more acid generating agents (C) generating an acid directly or indirectly by irradiation of any radiation selected from visible light, ultraviolet, excimer laser, electron beam, extreme ultraviolet (EUV), X-ray, and ion beam. The acid generating agent (C) is not particularly limited, and, for example, an acid generating agent described in International Publication No. WO2013/024778 can be used. The acid generating agent(C) can be used alone or in combination of two or more kinds. Among these acid generating agents, an acid generator having an aromatic ring is preferable from the viewpoint of heat resistance, and an acid generating agent having a structure represented by the following formula (8-1) or (8-2) is more preferable.

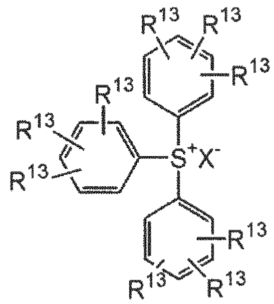

(8 − 1)

wherein $R^{13}$ may be the same or different, and are each independently a hydrogen atom, a linear, branched or cyclic alkyl group, a linear, branched or cyclic alkoxy group, a hydroxyl group, or a halogen atom, $X^-$ is an alkyl group, an aryl group, a sulfonic acid ion having a halogen-substituted alkyl group or a halogen-substituted aryl group, or a halide ion.

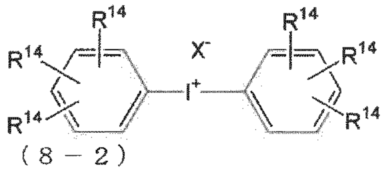

(8 − 2)

wherein $R^{14}$ may be the same or different, and each independently represents a hydrogen atom, a linear, branched or cyclic alkyl group, a linear, branched or cyclic alkoxy group, a hydroxyl group, or a halogen atom; and $X^-$ is as defined above.

The acid generating agent is even more preferably a compound in which $X^-$ in the formula (8-1) or (8-2) is a sulfonate ion having an aryl group or a halogen-substituted aryl group, and still more preferably a compound in which $X^-$ in the formula (8-1) or (8-2) is a sulfonate ion having an aryl group. Diphenyltrimethylphenylsulfonium p-toluenesulfonate, triphenylsulfonium p-toluenesulfonate, triphenylsulfonium trifluoromethanesulfonate, or triphenylsulfonium nonafluoromethanesulfonate is particularly preferable. Use of the acid generating agent can reduce LER.

The amount of the acid generating agent (C) used is preferably 0.001 to 49% by mass of the total mass of the solid components, more preferably 1 to 40% by mass, even more preferably 3 to 30% by mass, and particularly preferably 10 to 25% by mass. By using the acid generating agent (C) within the above range, a pattern profile with high sensitivity and low edge roughness tends to be obtained. In the present embodiment, the acid generation method is not limited as long as an acid is generated in the system. By using excimer laser instead of ultraviolet such as g-ray and i-ray, finer processing is possible, and also by using electron beam, extreme ultraviolet, X-ray or ion beam as a high energy ray, further finer processing is possible.

[Acid crosslinking agent (G)]

The resist composition of the present embodiment preferably contains one or more acid crosslinking agents (G). The acid crosslinking agent (G) is a compound capable of intramolecular or intermolecular crosslinking the component (A) in the presence of the acid generated from the acid generating agent (C). Examples of such an acid crosslinking agent (G) include a compound having one or more groups (hereinafter, referred to as "crosslinkable group") capable of crosslinking the component (A).

Examples of such a crosslinkable group can include (i) a hydroxyalkyl group such as a hydroxy (C1-C6 alkyl group), a C1-C6 alkoxy (C1-C6 alkyl group), and an acetoxy (C1-C6 alkyl group), or a group derived therefrom; (ii) a carbonyl group such as a formyl group and a carboxy (C1-C6 alkyl group), or a group derived therefrom; (iii) a nitrogenous group-containing group such as a dimethylaminomethyl group, a diethylaminomethyl group, a dimethylolaminomethyl group, a diethylolaminomethyl group, and a morpholinomethyl group; (iv) a glycidyl group-containing group such as a glycidyl ether group, a glycidyl ester group, and a glycidylamino group; (v) a group derived from an aromatic group such as an allyloxy (C1-C6 alkyl group) and an aralkyloxy (C1-C6 alkyl group) such as a benzyloxymethyl group and a benzoyloxymethyl group; and (vi) a polymerizable multiple bond-containing group such as a vinyl group and an isopropenyl group. Among those, the crosslinkable group of the acid crosslinking agent (G) is preferably a hydroxyalkyl group, an alkoxyalkyl group, or the like, and particularly preferably an alkoxymethyl group.

The acid crosslinking agent (G) having the crosslinkable group is not particularly limited, and, for example, an acid crosslinking agent described in International Publication No. WO2013/024778 can be used. The acid crosslinking

agent (G) can be used alone or in combination of two or more kinds.

The amount of the acid crosslinking agent (G) used is preferably 0.5 to 49% by mass of the total mass of the solid components, more preferably 0.5 to 40% by mass, even more preferably 1 to 30% by mass, and particularly preferably 2 to 20% by mass. When the content ratio of the above acid crosslinking agent (G) is 0.5% by mass or more, the inhibiting effect of the solubility of a resist film in an alkaline developer is improved, and a decrease in the film remaining rate and occurrence of swelling and meandering of a pattern tend to be inhibited. On the other hand, when the content is 49% by mass or less, a decrease in heat resistance as a resist tends to be inhibited.

[Acid diffusion controlling agent (E)]

The resist composition of the present embodiment may contain an acid diffusion controlling agent (E) having a function of controlling diffusion of an acid generated from an acid generating agent by radiation irradiation in a resist film to inhibit any unpreferable chemical reaction in an unexposed region or the like. By using such an acid diffusion controlling agent (E), the storage stability of a resist composition is improved. Also, along with the improvement of the resolution, the line width change of a resist pattern due to variation in the post exposure delay time before radiation irradiation and the post exposure delay time after radiation irradiation can be inhibited, and the composition has extremely excellent process stability. Examples of such an acid diffusion controlling agent (E) include, but not particularly limited to, a radiation degradable basic compound such as a nitrogen atom-containing basic compound, a basic sulfonium compound, and a basic iodonium compound.

The above acid diffusion controlling agent (E) is not particularly limited, and, for example, an acid diffusion controlling agent described in International Publication No. WO2013/024778 can be used. The acid diffusion controlling agent (E) can be used alone or in combination of two or more kinds.

The content of the acid diffusion controlling agent (E) is preferably 0.001 to 49% by mass of the total mass of the solid component, more preferably 0.01 to 10% by mass, even more preferably 0.01 to 5% by mass, and particularly preferably 0.01 to 3% by mass. Within the above range, a decrease in resolution, and deterioration of the pattern shape and the dimension fidelity or the like can be prevented. Moreover, even though the post exposure delay time from electron beam irradiation to heating after radiation irradiation becomes longer, the shape of the pattern upper layer portion is less likely to deteriorate. When the content is 10% by mass or less, a decrease in sensitivity, and developability of the unexposed portion or the like can be prevented. By using such an acid diffusion controlling agent, the storage stability of a resist composition improves, also along with improvement of the resolution, the line width change of a resist pattern due to variation in the post exposure delay time before radiation irradiation and the post exposure delay time after radiation irradiation can be inhibited, and the composition is extremely excellent process stability.

[Further component (F)]

To the resist composition of the present embodiment, optionally, as the further component (F) (also simply referred to as "optional component (F)"), one kind or two kinds or more of various additive agents such as a dissolution promoting agent, a dissolution controlling agent, a sensitizing agent, a surfactant, and an organic carboxylic acid or an oxo acid of phosphor or derivative thereof can be added.

[Dissolution promoting agent]

A dissolution promoting agent is a component having a function of increasing the solubility of a compound represented by the formula (1) in a developer to moderately increase the dissolution rate of the compound upon developing, when the solubility of the compound is too low and can be used as necessary. Examples of the above dissolution promoting agent include low molecular weight phenolic compounds, such as bisphenols and tris(hydroxyphenyl)methane. These dissolution promoting agents can be used alone or in combination of two or more kinds.

The content of the dissolution promoting agent, which is appropriately adjusted according to the kind of the compound to be used, is preferably 0 to 49% by mass of the total mass of the solid component, more preferably 0 to 5% by mass, even more preferably 0 to 1% by mass, and particularly preferably 0% by mass.

[Dissolution controlling agent]

The dissolution controlling agent is a component having a function of controlling the solubility of the compound represented by the formula (1) in a developer to moderately decrease the dissolution rate upon developing, when the solubility of the compound is too high. As such a dissolution controlling agent, the one which does not chemically change in steps such as calcination of resist coating, radiation irradiation, and development is preferable.

The dissolution controlling agent is not particularly limited, and examples include aromatic hydrocarbons such

as phenanthrene, anthracene, and acenaphthene; ketones such as acetophenone, benzophenone, and phenyl naphthyl ketone; and sulfones such as methyl phenyl sulfone, diphenyl sulfone, and dinaphthyl sulfone. These dissolution controlling agents can be used alone or in two or more kinds.

The content of the dissolution controlling agent, which is appropriately adjusted according to the kind of the compound to be used, is preferably 0 to 49% by mass of the total mass of the solid component, more preferably 0 to 5% by mass, even more preferably 0 to 1% by mass, and particularly preferably 0% by mass.

[Sensitizing Agent]

The sensitizing agent is a component having a function of absorbing irradiated radiation energy, transmitting the energy to the acid generating agent (C), and thereby increasing the acid production amount, and improving the apparent sensitivity of a resist. Such a sensitizing agent is not particularly limited, and examples include benzophenones, biacetyls, pyrenes, phenothiazines, and fluorenes. These sensitizing agents can be used alone or in two or more kinds.

The content of the sensitizing agent, which is appropriately adjusted according to the kind of the compound to be used, is preferably 0 to 49% by mass of the total mass of the solid component, more preferably 0 to 5% by mass, even more preferably 0 to 1% by mass, and particularly preferably 0% by mass.

[Surfactant]

The surfactant is a component having a function of improving coatability and striation of the resist composition, and developability of a resist or the like. Such a surfactant may be any of anionic, cationic, nonionic, or amphoteric surfactants. A preferable surfactant is a nonionic surfactant. The nonionic surfactant has a good affinity with a solvent used in production of resist compositions and more effects, and the above-mentioned effects become more remarkable. Examples of the nonionic surfactant include, but not particularly limited to, a polyoxyethylene higher alkyl ethers, polyoxyethylene higher alkyl phenyl ethers, and higher fatty acid diesters of polyethylene glycol. Examples of commercially available products include, hereinafter by trade name, EFTOP (manufactured by Jemco Inc.), MEGAFAC (manufactured by DIC Corporation), Fluorad (manufactured by Sumitomo 3M Limited), AsahiGuard, Surflon (hereinbefore, manufactured by Asahi Glass Co., Ltd.), Pepole (manufactured by Toho Chemical Industry Co., Ltd.), KP (manufactured by Shin-Etsu Chemical Co., Ltd.), and Polyflow (manufactured by Kyoeisha Chemical Co., Ltd.).

The content of the surfactant, which is appropriately adjusted according to the kind of the compound to be used, is preferably 0 to 49% by mass of the total mass of the solid component, more preferably 0 to 5% by mass, even more preferably 0 to 1% by mass, and particularly preferably 0% by mass.

[Organic carboxylic acid or oxo acid of phosphor or derivatives thereof]

For the purpose of prevention of sensitivity deterioration or improvement of a resist pattern shape and post exposure delay stability or the like, and as an additional optional component, the resist composition may contain an organic carboxylic acid or an oxo acid of phosphor or derivative thereof. The organic carboxylic acid or the oxo acid of phosphor or derivative thereof may be used in combination with the acid diffusion controlling agent, or may be used alone. The organic carboxylic acid is, for example, suitably malonic acid, citric acid, malic acid, succinic acid, benzoic acid, salicylic acid, or the like. Examples of the oxo acid of phosphor or derivative thereof include phosphoric acid or derivative thereof such as ester including phosphoric acid, di-n-butyl ester phosphate, and diphenyl ester phosphate; phosphonic acid or derivative thereof such as ester including phosphonic acid, dimethyl ester phosphonate, di-n-butyl ester phosphonate, phenylphosphonic acid, diphenyl ester phosphonate, and dibenzyl ester phosphonate; and phosphinic acid and derivative thereof such as ester including phosphinic acid and phenylphosphinic acid. Among these, phosphonic acid is particularly preferable.

The organic carboxylic acid or the oxo acid of phosphor or derivative thereof can be used alone or in combination of two or more kinds. The content of the organic carboxylic acid or the oxo acid of phosphor or derivative thereof, which is appropriately adjusted according to the kind of the compound to be used, is preferably 0 to 49% by mass of the total mass of the solid component, more preferably 0 to 5% by mass, even more preferably 0 to 1% by mass, and particularly preferably 0% by mass.

[Further additive agent other than above additive agents (dissolution promoting agent, dissolution controlling agent, sensitizing agent, surfactant, and organic carboxylic acid or oxo acid of phosphor or derivative thereof)]

Furthermore, the resist composition of the present embodiment can contain one kind or two kinds or more of additive agents other than the dissolution controlling agent, sensitizing agent, surfactant, and organic carboxylic acid or oxo acid of phosphor or derivative thereof optionally. Examples of such an additive agent include a dye, a pigment, and

an adhesion aid. For example, the composition contains the dye or the pigment, and thereby a latent image of the exposed portion is visualized and influence of halation upon exposure can be alleviated, which is preferable. The composition contains the adhesion aid, and thereby adhesiveness to a substrate can be improved, which is preferable. Furthermore, examples of other additive agent include a halation preventing agent, a storage stabilizing agent, a defoaming agent, and a shape improving agent. Specific examples thereof include 4-hydroxy-4'-methylchalkone.

In the resist composition of the present embodiment, the total content of the optional component (F) is 0 to 99% by mass of the total mass of the solid component, preferably 0 to 49% by mass, more preferably 0 to 10% by mass, even more preferably 0 to 5% by mass, still more preferably 0 to 1% by mass, and particularly preferably 0% by mass.

[Content ratio of each component in resist composition]

In the resist composition of the present embodiment, the content of the compound of the present embodiment and one or more substances selected from the compound group is not particularly limited, but is preferably 50 to 99.4% by mass of the total mass of the solid components (summation of solid components including the component (A), and optionally used components such as the acid generating agent (C), the acid crosslinking agent (G), the acid diffusion controlling agent (E), the further component (F) and the like, hereinafter the same), more preferably 55 to 90% by mass, even more preferably 60 to 80% by mass, and particularly preferably 60 to 70% by mass. In the case of the above content, resolution is further improved, and line edge roughness (LER) tends to be further decreased.

In the resist composition of the present embodiment, the content ratio of the component (A), the acid generating agent (C), the acid crosslinking agent (G), the acid diffusion controlling agent (E), and the optional component (F) (the component (A)/the acid generating agent (C)/the acid crosslinking agent (G)/the acid diffusion controlling agent (E)/the optional component (F)) is preferably 50 to 99.4% by mass/0.001 to 49% by mass/0.5 to 49% by mass/0.001 to 49% by mass/0 to 49% by mass, based on 100% by mass of the solid components of the resist composition, more preferably 55 to 90% by mass/1 to 40% by mass/0.5 to 40% by mass/0.01 to 10% by mass/0 to 5% by mass, even more preferably 60 to 80% by mass/3 to 30% by mass/1 to 30% by mass/0.01 to 5% by mass/0 to 1% by mass, and particularly preferably 60 to 70% by mass/10 to 25% by mass/2 to 20% by mass/0.01 to 3% by mass/0% by mass. The content ratio of each component is selected from each range so that the summation thereof is 100% by mass. Within the above content ratio, performance such as sensitivity, resolution, and developability tend to be excellent. The "solid components" refer to components except for the solvent. "100% by mass of the solid components" refer to 100% by mass of the components except for the solvent.

The resist composition of the present embodiment is generally prepared by dissolving each component in a solvent upon use into a homogeneous solution, and then optionally, filtering through a filter or the like with a pore diameter of about 0.2 $\mu$m, for example.

The resist composition of the present embodiment may optionally contain another resin other than the resin of the present embodiment. Examples of the other resin include, but not particularly limited to, a novolac resin, polyvinyl phenols, polyacrylic acid, polyvinyl alcohol, a styrene-maleic anhydride resin, and polymers containing an acrylic acid, vinyl alcohol or vinylphenol as a monomeric unit, and derivatives thereof. The content of the resin is not particularly limited and is appropriately adjusted according to the kind of the component (A) to be used, and is preferably 30 parts by mass or less per 100 parts by mass of the component (A), more preferably 10 parts by mass or less, even more preferably 5 parts by mass or less, and particularly preferably 0 part by mass.

[Physical properties and the like of resist composition]

The resist composition of the present embodiment can form an amorphous film by spin coating. Also, the resist composition of the present embodiment can be applied to a general semiconductor manufacturing process. Any of positive type and negative type resist patterns can be individually prepared depending on the kind of a developer to be used.

In the case of a positive type resist pattern, the dissolution rate of the amorphous film formed by spin coating with the resist composition of the present embodiment in a developer at 23°C is preferably 5 angstrom/sec or less, more preferably 0.05 to 5 angstrom/sec, and even more preferably 0.0005 to 5 angstrom/sec. When the dissolution rate is 5 angstrom/sec or less, the above portion is insoluble in a developer, and thus the amorphous film is easily formed into a resist. When the dissolution rate is 0.0005 angstrom/sec or more, the resolution may improve. It is presumed that this is because due to the change in the solubility before and after exposure of the component (A), contrast at the interface between the exposed portion being dissolved in a developer and the unexposed portion not being dissolved in a developer is increased. Also, the effects of reducing LER and defects are recognized.

In the case of using a negative type resist pattern, the dissolution rate of the amorphous film formed by spin coating with the resist composition of the present embodiment in a developer at 23°C is preferably 10 angstrom/sec or more. When the dissolution rate is 10 angstrom/sec or more, the amorphous film more easily dissolves in a developer,

and is more suitable for a resist. When the dissolution rate is 10 angstrom/sec or more, the resolution may improve. It is presumed that this is because the micro surface portion of the component (A) dissolves, and LER is reduced. Also, the effect of reducing defects is recognized.

The dissolution rate can be determined by immersing the amorphous film in a developer for a predetermined period of time at 23°C and then measuring the film thickness before and after immersion by a publicly known method such as visual, ellipsometric, or QCM method.

In the case of using a positive type resist pattern, the dissolution rate of the portion exposed by radiation such as KrF excimer laser, extreme ultraviolet, electron beam or X-ray, of the amorphous film formed by spin coating with the resist composition of the present embodiment, in a developer at 23°C is preferably 10 angstrom/sec or more. When the dissolution rate is 10 angstrom/sec or more, the amorphous film more easily dissolves in a developer, and is more suitable for a resist. When the amorphous film has a dissolution rate of 10 angstrom/sec or more, the resolution may improve. It is presumed that this is because the micro surface portion of the component (A) dissolves, and LER is reduced. Also, the effect of reducing defects is recognized.

In the case of a negative type resist pattern, the dissolution rate of the portion exposed by radiation such as KrF excimer laser, extreme ultraviolet, electron beam or X-ray, of the amorphous film formed by spin coating with the resist composition of the present embodiment, in a developer at 23°C is preferably 5 angstrom/sec or less, more preferably 0.05 to 5 angstrom/sec, and even more preferably 0.0005 to 5 angstrom/sec. When the dissolution rate is 5 angstrom/sec or less, the above portion is insoluble in a developer, and thus the amorphous film is easily formed into a resist. When the dissolution rate is 0.0005 angstrom/sec or more, the resolution may improve. It is presumed that this is because due to the change in the solubility before and after exposure of the component (A), contrast at the interface between the unexposed portion being dissolved in a developer and the exposed portion not being dissolved in a developer is increased. Also, the effects of reducing LER and defects are recognized.

[Radiation-sensitive composition]

The radiation-sensitive composition of the present embodiment is a radiation-sensitive composition containing one or more substances selected from the group consisting of the film forming materials of the present embodiment (component (A)) described above, an optically active diazonaphthoquinone compound (B), and a solvent, wherein the content of the solvent is 20 to 99% by mass based on 100% by mass in total of the radiation-sensitive composition.

The component (A) to be contained in the radiation-sensitive composition of the present embodiment is used in combination with the optically active diazonaphthoquinone compound (B) mentioned later and is useful as a base material for positive type resists that becomes a compound easily soluble in a developer by irradiation with g-ray, h-ray, i-ray, KrF excimer laser, ArF excimer laser, extreme ultraviolet, electron beam, or X-ray. Although the properties of the component (A) are not largely altered by irradiation of g-ray, h-ray, i-ray, KrF excimer laser, ArF excimer laser, extreme ultraviolet, electron beam, or X-ray, the optically active diazonaphthoquinone compound (B) poorly soluble in a developer is converted to an easily soluble compound so that a resist pattern can be formed in a development step.

Since the component (A) to be contained in the radiation-sensitive composition of the present embodiment is a relatively low molecular weight compound as shown in the formula (1), the obtained resist pattern has very small roughness. In the formula (1), at least one of $R_1$ to $R_3$ is preferably a group containing an iodine atom. In a case where the radiation-sensitive composition of the present embodiment contains the component (A) having a group containing an iodine atom, the ability to absorb radiation such as electron beam, extreme ultraviolet (EUV), or X-ray is increased. As a result, this enables the enhancement of the sensitivity, which is particularly preferable.

The glass transition temperature of the component (A) contained in the radiation-sensitive composition of the present embodiment is preferably 100°C or higher, more preferably 120°C or higher, even more preferably 140°C or higher, and particularly preferably 150°C or higher. The upper limit of the glass transition temperature of the component (A) is not particularly limited and is, for example, 400°C. When the glass transition temperature of the component (A) falls within the above range, the resulting radiation-sensitive composition has heat resistance capable of maintaining a pattern shape in a semiconductor lithography process, and the performance such as high resolution tends to be improved.

The heat of crystallization determined by the differential scanning calorimetry at the glass transition temperature of the component (A) contained in the radiation-sensitive composition of the present embodiment is preferably less than 20 J/g. (Crystallization temperature) - (Glass transition temperature) is preferably 70°C or more, more preferably 80°C or more, even more preferably 100°C or more, and particularly preferably 130°C or more. When the heat of crystallization is less than 20 J/g or (Crystallization temperature) - (Glass transition temperature) falls within the above range, there is a tendency that the radiation-sensitive composition easily forms an amorphous film by spin coating, can maintain film formability necessary for a resist over a long period, and can improve resolution.

In the present embodiment, the above heat of crystallization, crystallization temperature, and glass transition temperature can be determined by differential scanning calorimetry using "DSC/TA-50WS" manufactured by Shimadzu Corp. Specifically, for example, about 10 mg of a sample is placed in an unsealed container made of aluminum, and the

temperature is raised to the melting point or more at a temperature increase rate of 20°C/min in a nitrogen gas stream (50 mL/min). After quenching, again the temperature is raised to the melting point or more at a temperature increase rate of 20°C/min in a nitrogen gas stream (30 mL/min). After further quenching, again the temperature is raised to 400°C at a temperature increase rate of 20°C/min in a nitrogen gas stream (30 mL/min). The temperature at the middle point (where the specific heat is changed into the half) of steps in the baseline shifted in a step-like pattern is defined as the glass transition temperature (Tg). The temperature of the subsequently appearing exothermic peak is defined as the crystallization temperature. The heat is determined from the area of a region surrounded by the exothermic peak and the baseline and defined as the heat of crystallization.

The component (A) contained in the radiation-sensitive composition of the present embodiment is preferably low sublimable at 100°C or lower, preferably 120°C or lower, more preferably 130°C or lower, even more preferably 140°C or lower, and particularly preferably 150°C or lower at normal pressure. The low sublimability means that in thermogravimetry, weight reduction when the resist base material is kept at a predetermined temperature for 10 minutes is 10% or less, preferably 5% or less, more preferably 3% or less, even more preferably 1% or less, and particularly preferably 0.1% or less. The low sublimability can prevent an exposure apparatus from being contaminated by outgassing upon exposure. In addition, a good pattern shape with low roughness can be obtained.

The component (A) contained in the radiation-sensitive composition of the present embodiment dissolves at preferably 1% by mass or more, more preferably 5% by mass or more, and even more preferably 10% by mass or more at 23°C in a solvent that is selected from propylene glycol monomethyl ether acetate (PGMEA), propylene glycol monomethyl ether (PGME), cyclohexanone (CHN), cyclopentanone (CPN), 2-heptanone, anisole, butyl acetate, ethyl propionate, and ethyl lactate and exhibits the highest ability to dissolve the component (A). Particularly preferably, the component (A) dissolves at 20% by mass or more at 23°C in a solvent that is selected from PGMEA, PGME, and CHN and exhibits the highest ability to dissolve the resist base material (A). Particularly preferably, the component (A) dissolves at 20% by mass or more at 23°C in PGMEA. By satisfying the above conditions, the radiation-sensitive composition is easily used in a semiconductor production process at a full production scale.

[Optically active diazonaphthoquinone compound (B)]

The optically active diazonaphthoquinone compound (B) contained in the radiation-sensitive composition of the present embodiment is a diazonaphthoquinone substance including a polymer or non-polymer optically active diazonaphthoquinone compound and is not particularly limited as long as it is generally used as a photosensitive component (sensitizing agent) in positive type resist compositions. One kind or two or more kinds can be optionally selected and used.

Such a sensitizing agent is preferably, but not particularly limited to, a compound obtained by reacting naphthoquinonediazide sulfonic acid chloride, benzoquinonediazide sulfonic acid chloride, or the like with a low molecular weight compound or a high molecular weight compound having a functional group condensable with these acid chlorides. Herein, examples of the above functional group condensable with the acid chlorides include, but not particularly limited to, a hydroxyl group and an amino group. Particularly, a hydroxyl group is suitable. Examples of the compound containing a hydroxyl group condensable with the acid chlorides include, but not particularly limited to, hydroquinone, resorcin, hydroxybenzophenones such as 2,4-dihydroxybenzophenone, 2,3,4-trihydroxybenzophenone, 2,4,6-trihydroxybenzophenone, 2,4,4'-trihydroxybenzophenone, 2,3,4,4'-tetrahydroxybenzophenone, 2,2',4,4'-tetrahydroxybenzophenone, and 2,2',3,4,6'-pentahydroxybenzophenone, hydroxyphenylalkanes such as bis(2,4-dihydroxyphenyl)methane, bis(2,3,4-trihydroxyphenyl)methane, and bis(2,4-dihydroxyphenyl)propane, and hydroxytriphenylmethanes such as 4,4',3",4"-tetrahydroxy-3,5,3',5'-tetramethyltriphenylmethane and 4,4',2",3",4"-pentahydroxy-3,5,3',5'-tetramethyltriphenylmethane.

Preferable examples of the acid chloride such as naphthoquinonediazide sulfonic acid chloride or benzoquinonediazide sulfonic acid chloride include 1,2-naphthoquinonediazide-5-sulfonyl chloride and 1,2-naphthoquinonediazide-4-sulfonyl chloride.

The radiation-sensitive composition of the present embodiment is preferably prepared by, for example, dissolving each component in a solvent upon use into a homogeneous solution, and then optionally, filtering through a filter or the like with a pore diameter of about 0.2 μm, for example.

[Solvent]

Examples of the solvent that can be used in the radiation-sensitive composition of the present embodiment include, but not particularly limited to, propylene glycol monomethyl ether acetate, propylene glycol monomethyl ether, cyclohexanone, cyclopentanone, 2-heptanone, anisole, butyl acetate, ethyl propionate, and ethyl lactate. Among them, propylene glycol monomethyl ether acetate, propylene glycol monomethyl ether, or cyclohexanone is preferable. The solvent may be used alone or may be used in combination of two or more kinds.

The content of the solvent is 20 to 99% by mass based on 100% by mass in total of the radiation-sensitive

composition, preferably 50 to 99% by mass, more preferably 60 to 98% by mass, and particularly preferably 90 to 98% by mass.

The content of components except for the solvent (solid components) is 1 to 80% by mass based on 100% by mass in total of the radiation-sensitive composition, preferably 1 to 50% by mass, more preferably 2 to 40% by mass, particularly preferably 2 to 10% by mass.

[Properties of radiation-sensitive composition]

The radiation-sensitive composition of the present embodiment can form an amorphous film by spin coating. Also, the radiation-sensitive composition of the present embodiment can be applied to a general semiconductor manufacturing process. Any of positive type and negative type resist patterns can be individually prepared depending on the kind of a developer to be used.

In the case of a positive type resist pattern, the dissolution rate of the amorphous film formed by spin coating with the radiation-sensitive composition of the present embodiment in a developer at 23°C is preferably 5 angstrom/sec or less, more preferably 0.05 to 5 angstrom/sec, and even more preferably 0.0005 to 5 angstrom/sec. When the dissolution rate is 5 angstrom/sec or less, the above portion is insoluble in a developer, and thus the amorphous film is easily formed into a resist. When the dissolution rate is 0.0005 angstrom/sec or more, the resolution may improve. It is presumed that this is because due to the change in the solubility before and after exposure of the component (A), contrast at the interface between the exposed portion being dissolved in a developer and the unexposed portion not being dissolved in a developer is increased. Also, the effects of reducing LER and defects are recognized.

In the case of a negative type resist pattern, the dissolution rate of the amorphous film formed by spin coating with the radiation-sensitive composition of the present embodiment in a developer at 23°C is preferably 10 angstrom/sec or more. When the dissolution rate is 10 angstrom/sec or more, the amorphous film more easily dissolves in a developer, and is more suitable for a resist. When the dissolution rate is 10 angstrom/sec or more, the resolution may improve. It is presumed that this is because the micro surface portion of the component (A) dissolves, and LER is reduced. Also, the effect of reducing defects is recognized.

The dissolution rate can be determined by immersing the amorphous film in a developer for a predetermined period of time at 23°C and then measuring the film thickness before and after immersion by a publicly known method such as visual, ellipsometric, or QCM method.

In the case of a positive type resist pattern, the dissolution rate of the exposed portion after irradiation with radiation such as KrF excimer laser, extreme ultraviolet, electron beam or X-ray, or after heating at 20 to 500°C, of the amorphous film formed by spin coating with the radiation-sensitive composition of the present embodiment, in a developer at 23°C is preferably 10 angstrom/sec or more, more preferably 10 to 10000 angstrom/sec, and even more preferably 100 to 1000 angstrom/sec. When the dissolution rate is 10 angstrom/sec or more, the amorphous film more easily dissolves in a developer, and is more suitable for a resist. When the dissolution rate is 10000 angstrom/sec or less, the resolution may improve. It is presumed that this is because the micro surface portion of the component (A) dissolves, and LER is reduced. Also, the effect of reducing defects is recognized.

In the case of a negative type resist pattern, the dissolution rate of the exposed portion after irradiation with radiation such as KrF excimer laser, extreme ultraviolet, electron beam or X-ray, or after heating at 20 to 500°C, of the amorphous film formed by spin coating with the radiation-sensitive composition of the present embodiment, in a developer at 23°C is preferably 5 angstrom/sec or less, more preferably 0.05 to 5 angstrom/sec, and even more preferably 0.0005 to 5 angstrom/sec. When the dissolution rate is 5 angstrom/sec or less, the above portion is insoluble in a developer, and thus the amorphous film is easily formed into a resist. When the dissolution rate is 0.0005 angstrom/sec or more, the resolution may improve. It is presumed that this is because due to the change in the solubility before and after exposure of the component (A), contrast at the interface between the unexposed portion being dissolved in a developer and the exposed portion not being dissolved in a developer is increased. Also, the effects of reducing LER and defects are recognized.

[Content ratio of each component in radiation-sensitive composition]

In the radiation-sensitive composition of the present embodiment, the content of the component (A) is preferably 1 to 99% by mass of the total mass of the solid components (summation of the component (A), the optically active diazonaphthoquinone compound (B), and optionally used solid components such as further component (D), hereinafter the same), more preferably 5 to 95% by mass, even more preferably 10 to 90% by mass, and particularly preferably 25 to 75% by mass. When the content of the component (A) falls within the above range, the radiation-sensitive composition of the present embodiment tends to produce a pattern with high sensitivity and low roughness.

In the radiation-sensitive composition of the present embodiment, the content of the optically active diazonaphthoquinone compound (B) is preferably 1 to 99% by mass of the total mass of the solid components, more preferably 5

to 95% by mass, still more preferably 10 to 90% by mass, and particularly preferably 25 to 75% by mass. When the content of the optically active diazonaphthoquinone compound (B) falls within the above range, the radiation-sensitive composition of the present embodiment tends to produce a pattern with high sensitivity and low roughness.

[Further optional component (D)]

To the radiation-sensitive composition of the present embodiment, optionally, as a component other than the component (A) and the optically active diazonaphthoquinone compound (B), one kind or two kinds or more of various additive agents such as the above acid generating agent, acid crosslinking agent, acid diffusion controlling agent, dissolution promoting agent, dissolution controlling agent, sensitizing agent, surfactant, and organic carboxylic acid or oxo acid of phosphor or derivative thereof can be added. In the present specification, the further component (D) is also referred to as an "optional component (D)".

The content ratio of the component (A), the optically active diazonaphthoquinone compound (B), and the further optional component (D) that may be optionally contained in the radiation-sensitive composition ((A)/(B)/(D)) is preferably 1 to 99% by mass/99 to 1% by mass/0 to 98% by mass, based on 100% by mass of the solid components of the radiation-sensitive composition, more preferably 5 to 95% by mass/95 to 5% by mass/0 to 49% by mass, even more preferably 10 to 90% by mass/90 to 10% by mass/0 to 10% by mass, still more preferably 20 to 80% by mass/80 to 20% by mass/0 to 5% by mass, and particularly preferably 25 to 75% by mass/75 to 25% by mass/0% by mass.

The content ratio of each component is selected from each range so that the summation thereof is 100% by mass. When the content ratio of each component falls within the above range, the radiation-sensitive composition of the present embodiment tends to be excellent in performance such as sensitivity and resolution, in addition to roughness.

The radiation-sensitive composition of the present embodiment can optionally contain another resin other than the resin of the present embodiment. Examples of the resin include, but not particularly limited to, a novolac resin, polyvinyl phenols, polyacrylic acid, polyvinyl alcohol, a styrene-maleic anhydride resin, and polymers containing an acrylic acid, vinyl alcohol or vinylphenol as a monomeric unit, and derivatives thereof. The content of these resins, which is appropriately adjusted according to the kind of the component (A) to be used, is preferably 30 parts by mass or less per 100 parts by mass of the component (A), more preferably 10 parts by mass or less, even more preferably 5 parts by mass or less, and particularly preferably 0 part by mass.

[Method for producing amorphous film]

The method for producing an amorphous film according to the present embodiment comprises the step of forming an amorphous film on a substrate using the above radiation-sensitive composition.

[Resist pattern formation method using radiation-sensitive composition]

A resist pattern formation method using the radiation-sensitive composition of the present embodiment includes the steps of: forming a resist film on a substrate using the above radiation-sensitive composition; exposing at least a portion of the formed resist film; and developing the exposed resist film, thereby forming a resist pattern. Specifically, the same operation as in the following resist pattern formation method using the resist composition can be performed.

[Resist pattern formation method using resist composition]

A resist pattern formation method using the resist composition of the present embodiment includes the steps of: forming a resist film on a substrate using the above resist composition of the present embodiment; exposing at least a portion of the formed resist film; and developing the exposed resist film, thereby forming a resist pattern. The resist pattern according to the present embodiment can also be formed as an upper layer resist in a multilayer process.

Examples of the resist pattern formation method include, but not particularly limited to, the following methods. A resist film is formed by coating a conventionally publicly known substrate with the above resist composition of the present embodiment using a coating means such as spin coating, flow casting coating, and roll coating. The conventionally publicly known substrate is not particularly limited. For example, a substrate for electronic components, and the one having a predetermined wiring pattern formed thereon, or the like can be exemplified. More specific examples include a substrate made of a metal such as a silicon wafer, copper, chromium, iron and aluminum, and a glass substrate. Examples of a wiring pattern material include copper, aluminum, nickel, and gold. Also optionally, the substrate may be a substrate having an inorganic and/or organic film provided thereon. Examples of the inorganic film include an inorganic antireflection film (inorganic BARC). Examples of the organic film include an organic antireflection film (organic BARC). Surface treatment with hexamethylene disilazane or the like may be performed on the substrate.

Next, the substrate coated with the resist composition is heated as necessary. The heating conditions vary

according to the compounding composition of the resist composition, or the like, but are preferably 20 to 250°C, and more preferably 20 to 150°C. By heating, the adhesiveness of a resist to a substrate may improve, which is preferable. Then, the resist film is exposed to a desired pattern by any radiation selected from the group consisting of visible light, ultraviolet, excimer laser, electron beam, extreme ultraviolet (EUV), X-ray, and ion beam. The exposure conditions or the like are appropriately selected according to the compounding composition of the resist composition, or the like. In the present embodiment, in order to stably form a fine pattern with a high degree of accuracy in exposure, the resist film is preferably heated after radiation irradiation.

Next, by developing the exposed resist film in a developer, a predetermined resist pattern is formed. As the developer, a solvent having a solubility parameter (SP value) close to that of the component (A) to be used is preferably selected. A polar solvent such as a ketone-based solvent, an ester-based solvent, an alcohol-based solvent, an amide-based solvent, and an ether-based solvent; and a hydrocarbon-based solvent, or an alkaline aqueous solution can be used. Specifically, for example, those described in International Publication No. WO2013/024778 can be used.

A positive type resist pattern or a negative type resist pattern can be selectively made depending on the type of developers, but generally, in the case of polar solvents such as a ketone-based solvent, an ester-based solvent, an alcohol-based solvent, an amide-based solvent, and an ether-based solvent, and a hydrocarbon-based solvent, a negative resist pattern is obtained, and in the case of an alkaline aqueous solution, a positive resist pattern is obtained.

A plurality of above solvents may be mixed, or the solvent may be used by mixing the solvent with a solvent other than those described above or water within the range having performance. In order to sufficiently exhibit the effect of the present invention, the water content ratio as the whole developer is preferably less than 70% by mass and less than 50% by mass, more preferably less than 30% by mass, and even more preferably less than 10% by mass. Particularly preferably, the developer is substantially moisture free. That is, the content of the organic solvent in the developer is preferably 30% by mass or more and 100% by mass or less based on the total amount of the developer, preferably 50% by mass or more and 100% by mass or less, more preferably 70% by mass or more and 100% by mass or less, even more preferably 90% by mass or more and 100% by mass or less, and particularly preferably 95% by mass or more and 100% by mass or less.

Particularly, the developer containing at least one kind of solvent selected from a ketone-based solvent, an ester-based solvent, an alcohol-based solvent, an amide-based solvent, and an ether-based solvent tends to improve resist performance such as resolution and roughness of the resist pattern, which is preferable.

The vapor pressure of the developer is preferably 5 kPa or less at 20°C, more preferably 3 kPa or less, and particularly preferably 2 kPa or less. The evaporation of the developer on the substrate or in a developing cup is inhibited by setting the vapor pressure of the developer to 5 kPa or less, to improve temperature uniformity within a wafer surface, thereby resulting in improvement in dimensional uniformity within the wafer surface.

Specific examples of the developer having a vapor pressure of 5 kPa or less include those described in International Publication NO. WO2013/024778.

Specific examples of the developer having a vapor pressure of 2 kPa or less which is a particularly preferable range include those described in International Publication NO. WO2013/024778.

To the developer, a surfactant can be added in an appropriate amount, optionally. The surfactant is not particularly limited but, for example, an ionic or nonionic fluorine-based and/or silicon-based surfactant can be used. Examples of the fluorine-based and/or silicon-based surfactant include the surfactants described in Japanese Patent Application Laid-Open Nos. 62-36663, 61-226746, 61-226745, 62-170950, 63-34540, 7-230165, 8-62834, 9-54432, and 9-5988, and U.S. Pat. Nos. 5,405,720, 5,360,692, 5,529,881, 5,296,330, 5,436,098, 5,576,143, 5,294,511, and 5,824,451. The surfactant is preferably a nonionic surfactant. The nonionic surfactant is not particularly limited, but a fluorine-based surfactant or a silicon-based surfactant is more preferable.

The amount of the surfactant used is usually 0.001 to 5% by mass based on the total amount of the developer, preferably 0.005 to 2% by mass, and more preferably 0.01 to 0.5% by mass.

The development method is, for example, a method for dipping a substrate in a bath filled with a developer for a fixed time (dipping method), a method for raising a developer on a substrate surface by the effect of a surface tension and keeping it still for a fixed time, thereby conducting the development (puddle method), a method for spraying a developer on a substrate surface (spraying method), and a method for continuously ejecting a developer on a substrate rotating at a constant speed while scanning a developer ejecting nozzle at a constant rate (dynamic dispense method), or the like may be applied. The time for conducting the pattern development is not particularly limited, but is preferably 10 seconds to 90 seconds.

After the step of conducting development, a step of stopping the development by the replacement with another solvent may be practiced.

A step of rinsing the resist film with a rinsing solution containing an organic solvent is preferably provided after the development.

The rinsing solution used in the rinsing step after development is not particularly limited as long as the rinsing solution does not dissolve the resist pattern cured by crosslinking. A solution containing a general organic solvent or

water may be used as the rinsing solution. As the rinsing solution, a rinsing solution containing at least one kind of organic solvent selected from a hydrocarbon-based solvent, a ketone-based solvent, an ester-based solvent, an alcohol-based solvent, an amide-based solvent, and an ether-based solvent is preferably used. More preferably, after development, a step of rinsing the film by using a rinsing solution containing at least one kind of organic solvent selected from the group consisting of a ketone-based solvent, an ester-based solvent, an alcohol-based solvent and an amide-based solvent is conducted. Even more preferably, after development, a step of rinsing the film by using a rinsing solution containing an alcohol-based solvent or an ester-based solvent is conducted. Even more preferably, after development, a step of rinsing the film by using a rinsing solution containing a monohydric alcohol is conducted. Particularly preferably, after development, a step of rinsing the film by using a rinsing solution containing a monohydric alcohol having 5 or more carbon atoms is conducted. The time for rinsing the pattern is not particularly limited, but is preferably 10 seconds to 90 seconds.

Herein, examples of the monohydric alcohol used in the rinsing step after development are not particularly limited, and specific examples include monohydric alcohols described in International Publication No. WO 2013/024778.

A plurality of these components may be mixed, or the component may be used by mixing the component with an organic solvent other than those described above.

The water content ratio in the rinsing solution is preferably 10% by mass or less, more preferably 5% by mass or less, and even more preferably 3% by mass or less. By setting the water content ratio to 10% by mass or less, better development characteristics tends to be obtained.

The vapor pressure at 20°C of the rinsing solution used after development is preferably 0.05 kPa or more and 5 kPa or less, more preferably 0.1 kPa or more and 5 kPa or less, and even more preferably 0.12 kPa or more and 3 kPa or less. By setting the vapor pressure of the rinsing solution to 0.05 kPa or more and 5 kPa or less, the temperature uniformity in the wafer surface is enhanced and moreover, swelling due to permeation of the rinsing solution is further inhibited. As a result, the dimensional uniformity in the wafer surface is further improved.

The rinsing solution may also be used after adding an appropriate amount of a surfactant to the rinsing solution.

In the rinsing step, the wafer after development is rinsed using the organic solvent-containing rinsing solution. The method for rinsing treatment is not particularly limited. However, for example, a method for continuously ejecting a rinsing solution on a substrate spinning at a constant speed (spin coating method), a method for dipping a substrate in a bath filled with a rinsing solution for a fixed time (dipping method), and a method for spraying a rinsing solution on a substrate surface (spraying method), or the like can be applied. Above all, it is preferable to conduct the rinsing treatment by the spin coating method and after the rinsing, spin the substrate at a rotational speed of 2,000 rpm to 4,000 rpm, to remove the rinsing solution from the substrate surface.

After forming the resist pattern, a pattern wiring substrate is obtained by etching. Etching can be conducted by a publicly known method such as dry etching using plasma gas, and wet etching with an alkaline solution, a cupric chloride solution, and a ferric chloride solution or the like.

After forming the resist pattern, plating can also be conducted. Examples of the above plating method include copper plating, solder plating, nickel plating, and gold plating.

The remaining resist pattern after etching can be peeled by an organic solvent. Examples of the above organic solvent include PGMEA (propylene glycol monomethyl ether acetate), PGME (propylene glycol monomethyl ether), and EL (ethyl lactate). Examples of the above peeling method include a dipping method and a spraying method. A wiring substrate having a resist pattern formed thereon may be a multilayer wiring substrate, and may have a small diameter through hole.

In the present embodiment, the wiring substrate can also be formed by a method for forming a resist pattern, then depositing a metal in vacuum, and subsequently dissolving the resist pattern in a solution, i.e., a liftoff method.

[Underlayer film forming material for lithography]

The underlayer film forming material for lithography of the present embodiment contains one or more selected from the group consisting of the film forming materials of the present embodiment. The content of the component (A) contained in the material for underlayer film formation for lithography of the present embodiment in the underlayer film forming material for lithography is preferably 1 to 100% by mass, more preferably 10 to 100% by mass, even more preferably 50 to 100% by mass, particularly preferably 100% by mass, from the viewpoint of coatability and quality stability.

The underlayer film forming material for lithography of the present embodiment is applicable to a wet process and is excellent in heat resistance and etching resistance. Furthermore, the underlayer film forming material for lithography of the present embodiment employs the fore-mentioned triazine-based compound and can therefore form an underlayer film that is prevented from deteriorating during high temperature baking and is also excellent in etching resistance against oxygen plasma etching or the like. Moreover, the underlayer film forming material for lithography of the present embodiment is also excellent in adhesiveness to a resist layer and can therefore produce an excellent resist pattern. The underlayer film forming material for lithography of the present embodiment may contain an already known underlayer

film forming material for lithography or the like, within the range not deteriorating the effect of the present invention.

[Composition for underlayer film formation for lithography]

The composition for underlayer film formation for lithography of the present embodiment contains the above underlayer film forming material for lithography and a solvent.

[Solvent]

A publicly known solvent can be appropriately used as the solvent in the composition for underlayer film formation for lithography of the present embodiment as long as at least the above component (A) dissolves.
Specific examples of the solvent include, but not particularly limited to, those described in International Publication No. WO2013/024779. These solvents can be used alone as one kind or used in combination of two or more kinds. Among these solvents, cyclohexanone, propylene glycol monomethyl ether, propylene glycol monomethyl ether acetate, ethyl lactate, methyl hydroxyisobutyrate, or anisole is particularly preferable from the viewpoint of safety.
The content of the solvent is not particularly limited and is preferably 100 to 10,000 parts by mass per 100 parts by mass of the above underlayer film forming material, more preferably 200 to 5,000 parts by mass, and even more preferably 200 to 1,000 parts by mass, from the viewpoint of solubility and film formation.

[Crosslinking agent]

The composition for underlayer film formation for lithography of the present embodiment may contain a crosslinking agent, optionally, from the viewpoint of, for example, suppressing intermixing. The crosslinking agent that may be used in the present embodiment is not particularly limited, but a crosslinking agent described in, for example, International Publication No. WO 2013/024779 can be used. In the present embodiment, the crosslinking agent can be used alone or in combination of two or more kinds.
In the composition for underlayer film formation for lithography of the present embodiment, the content of the crosslinking agent is not particularly limited and is preferably 5 to 50 parts by mass per 100 parts by mass of the underlayer film forming material, and more preferably 10 to 40 parts by mass. By setting the content of the crosslinking agent in the above preferable range, a mixing event with a resist layer tends to be prevented. Also, an antireflection effect is enhanced, and film formability after crosslinking tends to be enhanced.

[Acid generating agent]

The composition for underlayer film formation for lithography of the present embodiment may contain an acid generating agent, optionally, from the viewpoint of, for example, further accelerating crosslinking reaction by heat. An acid generating agent that generates an acid by thermal decomposition, an acid generating agent that generates an acid by light irradiation, and the like are known, any of which can be used.
The acid generating agent is not particularly limited, and, for example, an acid generating agent described in International Publication No. WO2013/024779 can be used. In the present embodiment, the acid generating agent can be used alone or in combination of two or more kinds.
In the composition for underlayer film formation for lithography of the present embodiment, the content of the acid generating agent is not particularly limited and is preferably 0.1 to 50 parts by mass per 100 parts by mass of the underlayer film forming material, and more preferably 0.5 to 40 parts by mass. By setting the content of the acid generating agent in the above preferable range, crosslinking reaction tends to be enhanced by an increased amount of an acid generated. Also, a mixing event with a resist layer tends to be prevented.

[Basic compound]

The composition for underlayer film formation for lithography of the present embodiment may further contain a basic compound from the viewpoint of, for example, improving storage stability.
The basic compound plays a role as a quencher against acids in order to prevent crosslinking reaction from proceeding due to a trace amount of an acid generated by the acid generating agent. Examples of such a basic compound include, but not particularly limited to, primary, secondary or tertiary aliphatic amines, amine blends, aromatic amines, heterocyclic amines, nitrogen-containing compounds having a carboxy group, nitrogen-containing compounds having a sulfonyl group, nitrogen-containing compounds having a hydroxy group, nitrogen-containing compounds having a hydroxyphenyl group, alcoholic nitrogen-containing compounds, amide derivatives, and imide derivatives.
The basic compound used in the present embodiment is not particularly limited, and, for example, a basic

compound described in International Publication No. WO2013/024779 can be used. In the present embodiment, the basic compound can be used alone or in combination of two or more kinds.

In the composition for underlayer film formation for lithography of the present embodiment, the content of the basic compound is not particularly limited and is preferably 0.001 to 2 parts by mass per 100 parts by mass of the underlayer film forming material, and more preferably 0.01 to 1 parts by mass. By setting the content of the basic compound in the above preferable range, storage stability tends to be enhanced without excessively deteriorating crosslinking reaction.

[Further additive agent]

The composition for underlayer film formation for lithography of the present embodiment may also contain an additional resin and/or compound for the purpose of conferring thermosetting properties or controlling absorbance. Examples of such an additional resin and/or compound include, but not particularly limited to, naphthol resin, xylene resin naphthol-modified resin, phenol-modified resin of naphthalene resin, polyhydroxystyrene, dicyclopentadiene resin, resins containing (meth)acrylate, dimethacrylate, trimethacrylate, tetramethacrylate, a naphthalene ring such as vinylnaphthalene or polyacenaphthylene, a biphenyl ring such as phenanthrenequinone or fluorene, or a heterocyclic ring having a heteroatom such as thiophene or indene, and resins containing no aromatic ring; and resins or compounds containing an alicyclic structure, such as rosin-based resin, cyclodextrin, adamantine(poly)ol, tricyclodecane(poly)ol, and derivatives thereof. The composition for underlayer film formation for lithography of the present embodiment may further contain a publicly known additive agent. Examples of the above publicly known additive agent include, but not limited to, ultraviolet absorbers, surfactants, colorants, and nonionic surfactants.

[Underlayer film for lithography formation method]

The method for forming an underlayer film for lithography according to the present embodiment includes the step of forming an underlayer film on a substrate using the composition for underlayer film formation for lithography of the present embodiment.

[Resist pattern formation method using composition for underlayer film formation for lithography]

A resist pattern formation method using the composition for underlayer film formation for lithography of the present embodiment includes the steps of: forming an underlayer film on a substrate using the composition for underlayer film formation for lithography of the present embodiment (step (A-1)); forming at least one photoresist layer on the underlayer film (step (A-2)); and irradiating a predetermined region of the photoresist layer with radiation for development, thereby forming a resist pattern (step (A-3)).

[Circuit pattern formation method using composition for underlayer film formation for lithography]

A circuit pattern formation method using the composition for underlayer film formation for lithography of the present embodiment includes the steps of: forming an underlayer film on a substrate using the composition for underlayer film formation for lithography of the present embodiment (step (B-1)); forming an intermediate layer film on the underlayer film using a resist intermediate layer film material containing a silicon atom (step (B-2)); forming at least one photoresist layer on the intermediate layer film (step (B-3)); after the step (B-3), irradiating a predetermined region of the photoresist layer with radiation for development, thereby forming a resist pattern (step (B-4)); after the step (B-4), etching the intermediate layer film with the resist pattern as a mask, thereby forming an intermediate layer film pattern (step (B-5)); etching the underlayer film with the obtained intermediate layer film pattern as an etching mask, thereby forming an underlayer film pattern (step (B-6)); and etching the substrate with the obtained underlayer film pattern as an etching mask, thereby forming a pattern on the substrate (step (B-7)).

The underlayer film for lithography of the present embodiment is not particularly limited by its formation method as long as it is formed from the composition for underlayer film formation for lithography of the present embodiment. A publicly known approach can be applied thereto. The underlayer film can be formed by, for example, applying the composition for underlayer film formation for lithography of the present embodiment onto a substrate by a publicly known coating method or printing method such as spin coating or screen printing, and then removing an organic solvent by volatilization or the like.

It is preferable to perform baking in the formation of the underlayer film, for preventing a mixing event with an upper layer resist while accelerating crosslinking reaction. In this case, the baking temperature is not particularly limited and is preferably in the range of 80 to 450°C, and more preferably 200 to 400°C. The baking time is not particularly limited and is preferably in the range of 10 to 300 seconds. The thickness of the underlayer film can be appropriately

selected according to required performance and is not particularly limited, but is usually preferably about 30 to 20,000 nm, and more preferably 50 to 15,000 nm.

After preparing the underlayer film, it is preferable to prepare a silicon-containing resist layer or a usual single-layer resist made of hydrocarbon thereon in the case of a two-layer process, and to prepare a silicon-containing intermediate layer thereon and further a silicon-free single-layer resist layer thereon in the case of a three-layer process. In this case, a publicly known photoresist material can be used for forming this resist layer.

After preparing the underlayer film on the substrate, a silicon-containing resist layer or a usual single-layer resist made of hydrocarbon thereon can be prepared on the underlayer film in the case of a two-layer process. In the case of a three-layer process, a silicon-containing intermediate layer can be prepared on the underlayer film, and a silicon-free single-layer resist layer can be further prepared on the silicon-containing intermediate layer. In these cases, a publicly known photoresist material can be appropriately selected and used for forming the resist layer, without particular limitations.

For the silicon-containing resist material for a two-layer process, a silicon atom-containing polymer such as a polysilsesquioxane derivative or a vinylsilane derivative is used as a base polymer, and a positive type photoresist material further containing an organic solvent, an acid generating agent, and optionally, a basic compound or the like is preferably used, from the viewpoint of oxygen gas etching resistance. Herein, a publicly known polymer that is used in this kind of resist material can be used as the silicon atom-containing polymer.

A polysilsesquioxane-based intermediate layer is preferably used as the silicon-containing intermediate layer for a three-layer process. By imparting effects as an antireflection film to the intermediate layer, there is a tendency that reflection can be effectively suppressed. For example, use of a material containing a large amount of an aromatic group and having high substrate etching resistance as the underlayer film in a process for exposure at 193 nm tends to increase a k value and enhance substrate reflection. However, the intermediate layer suppresses the reflection so that the substrate reflection can be 0.5% or less. The intermediate layer having such an antireflection effect is not limited, and for example, polysilsesquioxane that crosslinks by an acid or heat in which a light absorbing group having a phenyl group or a silicon-silicon bond is introduced is preferably used for exposure at 193 nm.

Alternatively, an intermediate layer formed by chemical vapour deposition (CVD) may be used. The intermediate layer highly effective as an antireflection film prepared by CVD is not limited, and, for example, a SiON film is known. In general, the formation of an intermediate layer by a wet process such as spin coating or screen printing is more convenient and more advantageous in cost, as compared with CVD. The upper layer resist for a three-layer process may be positive type or negative type, and the same as a single-layer resist generally used can be used.

The underlayer film according to the present embodiment can also be used as an antireflection film for usual single-layer resists or an underlying material for suppression of pattern collapse. The underlayer film of the present embodiment is excellent in etching resistance for an underlying process and can be expected to also function as a hard mask for an underlying process.

In the case of forming a resist layer from the above photoresist material, a wet process such as spin coating or screen printing is preferably used, as in the case of forming the above underlayer film. After coating with the resist material by spin coating or the like, prebaking is generally performed. This prebaking is preferably performed at 80 to 180°C in the range of 10 to 300 seconds. Then, exposure, post-exposure baking (PEB), and development can be performed according to a conventional method to obtain a resist pattern. The thickness of the resist film is not particularly limited and is preferably 30 to 500 nm, and more preferably 50 to 400 nm in general.

The exposure light can be appropriately selected and used according to the photoresist material to be used. General examples thereof include a high energy ray having a wavelength of 300 nm or less, specifically, excimer laser of 248 nm, 193 nm, or 157 nm, soft x-ray of 3 to 20 nm, electron beam, and X-ray.

In a resist pattern formed by the above method, pattern collapse is suppressed by the underlayer film according to the present embodiment. Therefore, use of the underlayer film according to the present embodiment can produce a finer pattern and allows reducing an exposure amount necessary for obtaining the resist pattern.

Next, etching is performed with the obtained resist pattern as a mask. Gas etching is preferably used as the etching of the underlayer film in a two-layer process. The gas etching is preferably etching using oxygen gas. In addition to oxygen gas, an inert gas such as He or Ar, or CO, $CO_2$, $NH_3$, $SO_2$, $N_2$, $NO_2$, or $H_2$ gas may be added. Alternatively, the gas etching may be performed with CO, $CO_2$, $NH_3$, $N_2$, $NO_2$, or $H_2$ gas without the use of oxygen gas. Particularly, the latter gas is preferably used for side wall protection in order to prevent the undercut of pattern side walls.

On the other hand, gas etching is also preferably used as the etching of the intermediate layer in a three-layer process. The same gas etching as described in the above two-layer process is applicable. Particularly, it is preferable to process the intermediate layer in a three-layer process by using chlorofluorocarbon-based gas and using the resist pattern as a mask. Then, as mentioned above, for example, the underlayer film can be processed by oxygen gas etching with the intermediate layer pattern as a mask.

Herein, in the case of forming an inorganic hard mask intermediate layer film as the intermediate layer, a silicon oxide film, a silicon nitride film, or a silicon oxynitride film (SiON film) is formed by CVD, ALD, or the like. A method for

forming the nitride film is not limited, and, for example, a method described in Japanese Patent Laid-Open No. 2002-334869 (Patent Literature 6) or WO2004/066377 (Patent Literature 7) can be used. Although a photoresist film can be formed directly on such an intermediate layer film, an organic antireflection film (BARC) may be formed on the intermediate layer film by spin coating and a photoresist film may be formed thereon.

A polysilsesquioxane-based intermediate layer is preferably used as the intermediate layer. By imparting effects as an antireflection film to the resist intermediate layer film, there is a tendency that reflection can be effectively suppressed. A specific material for the polysilsesquioxane-based intermediate layer is not limited, and, for example, a material described in Japanese Patent Laid-Open No. 2007-226170 (Patent Literature 8) or Japanese Patent Laid-Open No. 2007-226204 (Patent Literature 9) can be used.

The subsequent etching of the substrate can also be performed by a conventional method. For example, the substrate made of $SiO_2$ or SiN can be etched mainly using chlorofluorocarbon-based gas, and the substrate made of p-Si, Al, or W can be etched mainly using chlorine- or bromine-based gas. In the case of etching the substrate with chlorofluorocarbon-based gas, the silicon-containing resist of the two-layer resist process or the silicon-containing intermediate layer of the three-layer process is peeled at the same time with substrate processing. On the other hand, in the case of etching the substrate with chlorine- or bromine-based gas, the silicon-containing resist layer or the silicon-containing intermediate layer is separately peeled and in general, peeled by dry etching using chlorofluorocarbon-based gas after substrate processing.

A feature of the underlayer film according to the present embodiment is that it is excellent in etching resistance of these substrates. The substrate can be appropriately selected from publicly known ones and used and is not particularly limited. Examples thereof include Si, $\alpha$-Si, p-Si, $SiO_2$, SiN, SiON, W, TiN, and Al. The substrate may be a laminate having a film to be processed (substrate to be processed) on a base material (support). Examples of such a film to be processed include various low-k films such as Si, $SiO_2$, SiON, SiN, p-Si, $\alpha$-Si, W, W-Si, Al, Cu, and Al-Si, and stopper films thereof. A material different from that for the base material (support) is generally used. The thickness of the substrate to be processed or the film to be processed is not particularly limited and is preferably 50 to 1,000,000 nm, and more preferably 75 to 500,000 nm.

[Permanent film for resist]

The above resist composition can also be used to prepare a permanent film for a resist. The permanent film for a resist prepared by coating with the above resist composition is suitable as a permanent film that also remains in a final product, optionally, after formation of a resist pattern. Specific examples of the permanent film include, but not limited to, in relation to semiconductor devices, solder resists, package materials, underfill materials, package adhesive layers for circuit elements and the like, and adhesive layers between integrated circuit elements and circuit substrates, and in relation to thin displays, thin film transistor protecting films, liquid crystal color filter protecting films, black matrixes, and spacers. Particularly, the permanent film made of the above resist composition is excellent in heat resistance and humidity resistance and furthermore, also has the excellent advantage that contamination by sublimable components is reduced. Particularly, for a display material, a material that achieves all of high sensitivity, high heat resistance, and hygroscopic reliability with reduced deterioration in image quality due to significant contamination can be obtained.

In the case of using the above resist composition for a permanent film for a resist, a curing agent as well as, optionally, various additive agents such as other resins, a surfactant, a dye, a filler, a crosslinking agent, and a dissolution promoting agent can be added and dissolved in an organic solvent to prepare a composition for permanent films for a resist.

The above film forming composition for lithography or resist composition can be prepared by adding each of the above components and mixing them using a stirrer or the like. When the above composition for resist underlayer films or resist composition contains a filler or a pigment, the compositions can be prepared by dispersion or mixing using a dispersion apparatus such as a dissolver, a homogenizer, and a three-roll mill.

[Method for purifying triazine-based compounds]

The method for purifying the triazine-based compound represented by the formula (1) comprises the steps of: obtaining a solution (S) by dissolving the compound of the present embodiment; and extracting impurities in the compound by bringing the obtained solution (S) into contact with an acidic aqueous solution (a first extraction step), wherein the solvent used in the step of obtaining the solution (S) contains an organic solvent immiscible with water.

According to the purification method of the present embodiment, the contents of various metals that may be contained in triazine-based compound described above may be reduced.

More specifically, in the purification method of the present embodiment, the triazine-based compound is dissolved in an organic solvent immiscible with water to obtain the solution (S), and further, extraction treatment can be carried out by bringing the solution (S) into contact with an acidic aqueous solution. Thereby, metals contained in the solution (S) containing the triazine-based compound of the present embodiment are transferred to the aqueous phase,

then the organic phase and the aqueous phase are separated, and thus the triazine-based compound of the present embodiment having a reduced metal content can be obtained.

The triazine-based compound of the present embodiment used in the purification method of the present embodiment may be alone, or may be a mixture of two or more kinds. Also, the triazine-based compound of the present embodiment may contain various surfactants, various crosslinking agents, various acid generating agents, various stabilizers, and the like.

The solvent immiscible with water used in the purification method of the present embodiment is not particularly limited, but is preferably an organic solvent that is safely applicable to semiconductor manufacturing processes, and specifically it is an organic solvent having a solubility in water at room temperature of less than 30%, and more preferably is an organic solvent having a solubility of less than 20% and particularly preferably less than 10%. The amount of the organic solvent used is preferably 1 to 100 times the mass of the triazine-based compound of the present embodiment.

Specific examples of the solvent immiscible with water include, but not limited to, those described in International Publication No. WO2015/080240. Among these, toluene, 2-heptanone, cyclohexanone, cyclopentanone, methyl isobutyl ketone, propylene glycol monomethyl ether acetate, ethyl acetate, and the like are preferable, methyl isobutyl ketone, ethyl acetate, cyclohexanone, and propylene glycol monomethyl ether acetate are more preferable, and methyl isobutyl ketone and ethyl acetate are further preferable. Methyl isobutyl ketone, ethyl acetate, and the like have relatively high saturation solubility for the triazine-based compound of the present embodiment and a relatively low boiling point, and it is thus possible to reduce the load in the case of industrially distilling off the solvent and in the step of removing the solvent by drying. These solvents can be each used alone, and can be used as a mixture of two or more kinds.

Examples of the acidic aqueous solution used in the purification method of the present embodiment include, but not particularly limited to, those described in International Publication No. WO2015/080240. These acidic aqueous solutions can be each used alone, and can be also used as a combination of two or more kinds. Among these acidic aqueous solutions, aqueous solutions of one or more mineral acids selected from the group consisting of hydrochloric acid, sulfuric acid, nitric acid, and phosphoric acid, or aqueous solutions of one or more organic acids selected from the group consisting of acetic acid, propionic acid, oxalic acid, malonic acid, succinic acid, fumaric acid, maleic acid, tartaric acid, citric acid, methanesulfonic acid, phenolsulfonic acid, p-toluenesulfonic acid, and trifluoroacetic acid are preferable, aqueous solutions of sulfuric acid, nitric acid, and carboxylic acids such as acetic acid, oxalic acid, tartaric acid, and citric acid are more preferable, aqueous solutions of sulfuric acid, oxalic acid, tartaric acid, and citric acid are even more preferable, and an aqueous solution of oxalic acid is still more preferable. It is considered that polyvalent carboxylic acids such as oxalic acid, tartaric acid, and citric acid coordinate with metal ions and provide a chelating effect, and thus tend to be capable of more effectively removing metals. As for water used herein, it is preferable to use water, the metal content of which is small, such as ion exchanged water, according to the purpose of the purification method of the present embodiment.

The pH of the acidic aqueous solution used in the purification method of the present embodiment is not particularly limited, but it is preferable to regulate the acidity of the aqueous solution in consideration of an influence on the triazine-based compound of the present embodiment. Normally, the pH range is about 0 to 5, and is preferably about pH 0 to 3.

The amount of the acidic aqueous solution used in the purification method of the present embodiment is not particularly limited, but it is preferable to regulate the amount from the viewpoint of reducing the number of extraction operations for removing metals and from the viewpoint of ensuring operability in consideration of the overall amount of fluid. From the above viewpoints, the amount of the acidic aqueous solution used is preferably 10 to 200 parts by mass, and more preferably 20 to 100 parts by mass, based on 100 parts by mass of the solution (S) .

In the purification method of the present embodiment, by bringing an acidic aqueous solution as described above into contact with the solution (S) containing the triazine-based compound of the present embodiment and the solvent immiscible with water, metals can be extracted from the triazine-based compound in the solution (S).

In the purification method of the present embodiment, it is preferable that the solution (S) further contains an organic solvent immiscible with water. When an organic solvent immiscible with water is contained, there is a tendency that the amount of the triazine-based compound of the present embodiment charged can be increased, also the fluid separability is improved, and purification can be carried out at a high reaction vessel efficiency. The method for adding the organic solvent immiscible with water is not particularly limited. For example, any of a method involving adding it to the organic solvent-containing solution in advance, a method involving adding it to water or the acidic aqueous solution in advance, and a method involving adding it after bringing the organic solvent-containing solution into contact with water or the acidic aqueous solution. Among these, the method involving adding it to the organic solvent-containing solution in advance is preferable in terms of the workability of operations and the ease of managing the amount.

The organic solvent immiscible with water used in the purification method of the present embodiment is not particularly limited, but is preferably an organic solvent that is safely applicable to semiconductor manufacturing processes. The amount of the organic solvent used immiscible with water is not particularly limited as long as the solution phase and the aqueous phase separate, but is preferably 0.1 to 100 times, more preferably 0.1 to 50 times, and even

more preferably 0.1 to 20 times the mass of the triazine-based compound and/or the resin of the present embodiment.

Specific examples of the organic solvent used immiscible with water in the purification method of the present embodiment include, but not limited to, those described in International Publication No. WO2015/080240. Among these, N-methylpyrrolidone, propylene glycol monomethyl ether, and the like are preferable, and N-methylpyrrolidone and propylene glycol monomethyl ether are more preferable. These solvents can be each used alone, and can be used as a mixture of two or more kinds.

The temperature when extraction treatment is carried out is generally in the range of 20 to 90°C, and preferably 30 to 80°C. The extraction operation is carried out, for example, by thoroughly mixing the solution (S) and the acidic aqueous solution by stirring or the like and then leaving the obtained mixed solution to stand still. Thereby, metals contained in the solution containing the triazine-based compound of the present embodiment and the organic solvents are transferred to the aqueous phase. Also, by this operation, the acidity of the solution is lowered, and the degradation of the triazine-based compound of the present embodiment can be suppressed.

By being left to stand still, the mixed solution is separated into an aqueous phase and a solution phase containing the triazine-based compound of the present embodiment and the solvents, and thus the solution phase containing the triazine-based compound of the present embodiment and the solvents is recovered by decantation or the like. The time for leaving the mixed solution to stand still is not particularly limited, but it is preferable to regulate the time for leaving the mixed solution to stand still from the viewpoint of attaining good separation of the solution phase containing the solvents and the aqueous phase. Normally, the time for leaving the mixed solution to stand still is 1 minute or longer, preferably 10 minutes or longer, and more preferably 30 minutes or longer. While the extraction treatment may be carried out once, it is effective to repeat mixing, leaving-to-stand-still, and separating operations multiple times.

It is preferable that the purification method of the present embodiment includes the step of extracting impurities in the triazine-based compound by further bringing the solution phase containing the triazine-based compound into contact with water after the first extraction step (the second extraction step). Specifically, for example, it is preferable that after the above extraction treatment is carried out using an acidic aqueous solution, the solution phase that is extracted and recovered from the aqueous solution and that contains the triazine-based compound of the present embodiment and the solvents is further subjected to extraction treatment with water. The above extraction treatment with water is not particularly limited, and can be carried out, for example, by thoroughly mixing the solution phase and water by stirring or the like and then leaving the obtained mixed solution to stand still. The mixed solution after being left to stand still is separated into an aqueous phase and a solution phase containing the triazine-based compound of the present embodiment and the solvents, and thus the solution phase containing the triazine-based compound of the present embodiment and the solvents can be recovered by decantation or the like.

Water used herein is preferably water, the metal content of which is small, such as ion exchanged water, according to the purpose of the present embodiment. While the extraction treatment may be carried out once, it is effective to repeat mixing, leaving-to-stand-still, and separating operations multiple times. The proportions of both used in the extraction treatment and temperature, time, and other conditions are not particularly limited, and may be the same as those of the previous contact treatment with the acidic aqueous solution.

Water that is possibly present in the thus-obtained solution containing the triazine-based compound of the present embodiment and the solvents can be easily removed by performing vacuum distillation or a like operation. Also, optionally, the concentration of the triazine-based compound of the present embodiment can be regulated to be any concentration by adding a solvent to the above solution.

The method for isolating the triazine-based compound of the present embodiment from the obtained solution containing the triazine-based compound of the present embodiment and the solvents is not particularly limited, and publicly known methods can be carried out, such as reduced-pressure removal, separation by reprecipitation, and a combination thereof. Publicly known treatments such as concentration operation, filtration operation, centrifugation operation, and drying operation can further be carried out optionally.

Examples

The present embodiment will be more specifically described with reference to the examples below. However, the present embodiment is not particularly limited to these examples.

[Evaluation of heat resistance]

EXSTAR 6000 TG-DTA apparatus manufactured by SII NanoTechnology Inc. was used. About 5 mg of a sample was placed in an unsealed container made of aluminum, and the temperature was raised to 500°C at a temperature increase rate of 10°C/min in a nitrogen gas stream (100 ml/min) . Thus, the thermal weight loss was measured and evaluated according to the following criteria.

From the practical viewpoint, it is preferable to be evaluated as A or B below. Evaluation of A or B indicates

high heat resistance and applicability to high temperature baking.

<Evaluation criteria>

A: Thermal weight loss at 400°C is less than 10%
B: Thermal weight loss at 400°C is 10% to 25%
C: Thermal weight loss at 400°C is more than 25%

[Evaluation of solubility]

The compound was added to a 50 mL screw bottle and stirred at 23°C for 1 hour using a magnetic stirrer. Then, the amount of the compound dissolved in orthoxylene (OX) was measured, and the result was evaluated according to the following criteria.

From the practical viewpoint, it is preferable to be evaluated as A or B below. Evaluation of A or B indicates high storage stability in a solution state, and applicability to a semiconductor microfabrication process.

<Evaluation criteria>

A: 15% by mass or more
B: 10 mass% or more and less than 15 mass%
C: less than 10% by mass

[Example 1]

A triazine compound having a structure represented by the following formula (LA-F70 manufactured by ADEKA Co., Ltd.) alone was used as a material for film formation for lithography.

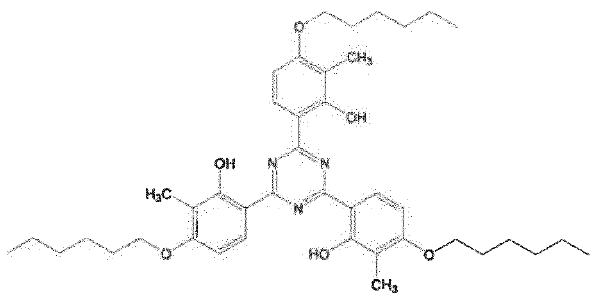

LA-F70

As a result of thermogravimetry, the amount of thermal loss at 400°C of the obtained material for film formation for lithography was less than 10% (evaluation A). As a result of evaluating the solubility in OX, it was evaluated that the solubility was 10 mass% or more and less than 15 mass% (evaluation B), and the obtained material for film formation for lithography had sufficient solubility.

90 parts by mass of OX as a solvent was added to 10 parts by mass of the material for film formation for lithography, and stirred at room temperature for at least 3 hours or more with a stirrer to prepare a film forming composition for lithography.

[Example 2]

A triazine compound having a structure represented by the following formula (TINUVIN 460 manufactured by BASF Corporation) alone was used as a material for film formation for lithography.

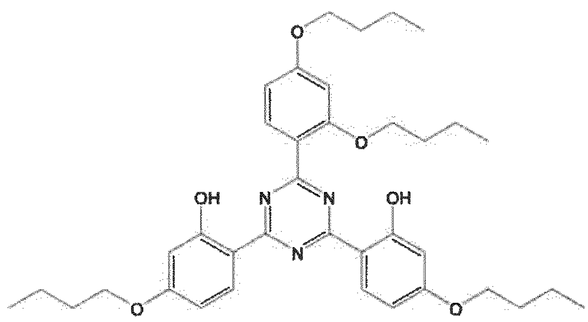

TINUVIN460

As a result of thermogravimetry, the amount of thermal loss at 400°C of the obtained material for film formation for lithography was 10% to 25% (evaluation B). As a result of evaluating the solubility in OX, it was evaluated that the solubility was 15 mass% or more (evaluation A), and the obtained material for film formation for lithography had excellent solubility.

90 parts by mass of OX as a solvent was added to 10 parts by mass of the material for film formation for lithography, and stirred at room temperature for at least 3 hours or more with a stirrer to prepare a film forming composition for lithography.

[Comparative Example 1]

A four necked flask (internal capacity: 10 L) equipped with a Dimroth condenser tube, a thermometer, and a stirring blade and having a detachable bottom was prepared. To this four necked flask, 1.09 kg (7 mol) of 1,5-dimethylnaphthalene (manufactured by Mitsubishi Gas Chemical Company, Inc.), 2.1 kg (28 mol as formaldehyde) of 40% by mass of an aqueous formalin solution (manufactured by Mitsubishi Gas Chemical Company, Inc.), and 0.97 mL of 98% by mass of sulfuric acid (manufactured by Kanto Chemical Co., Inc.) were added in a nitrogen stream, and the mixture was reacted for 7 hours while refluxed at 100°C at normal pressure. Subsequently, 1.8 kg of ethylbenzene (special grade reagent manufactured by Wako Pure Chemical Industries, Ltd.) was added as a diluting solvent to the reaction solution, and the mixture was left to stand still, followed by removal of an aqueous phase as a lower phase. Neutralization and washing with water were further performed, and ethylbenzene and unreacted 1,5-dimethylnaphthalene were distilled off under reduced pressure to obtain 1.25 kg of a light brown solid dimethylnaphthalene formaldehyde resin.

Regarding the molecular weight of the obtained dimethylnaphthalene formaldehyde, the number average molecular weight (Mn) was 562, the weight molecular weight (Mw) was 1168, and the dispersion degree (Mw/Mn) was 2.08.

Then, a four necked flask (internal capacity: 0.5 L) equipped with a Dimroth condenser tube, a thermometer, and a stirring blade was prepared. To this four necked flask, 100 g (0.51 mol) of the dimethylnaphthalene formaldehyde resin thus obtained, and 0.05 g of p-toluenesulfonic acid were added in a nitrogen stream, and the temperature was raised to 190°C at which the mixture was then heated for 2 hours, followed by stirring. Subsequently, 52.0 g (0.36 mol) of 1-naphthol was further added thereto, the temperature was further elevated to 220°C, and the mixture was reacted for 2 hours. After dilution with a solvent, neutralization and washing with water were performed, and the solvent was distilled off under reduced pressure to obtain 126.1 g of a modified resin (CR-1) as a black-brown solid.

The obtained resin (CR-1) had Mn: 885, Mw: 2220, and Mw/Mn: 4.17.

As a result of thermogravimetry (TG), the amount of thermal loss at 400°C of the obtained resin was more than 25% (evaluation C). Therefore, it was evaluated that application to high temperature baking is difficult. As a result of evaluating the solubility in OX, it was evaluated that the solubility was 15% by mass or more (evaluation A), and thus evaluated that the obtained material had excellent solubility.

The above Mn, Mw, and Mw/Mn were measured by performing gel permeation chromatography (GPC) analysis under the following conditions to determine the molecular weight in terms of polystyrene.

Apparatus: Shodex GPC-101 model (manufactured by Showa Denko K.K.)
Column: KF-80M x 3
Eluent: 1 mL/min THF
Temperature: 40°C

(Heat resistance evaluation)

As is evident from Table 1, it was able to be confirmed that the composition for film formation for lithography

of Example 1 has good heat resistance.

[Table 1]

| | Triazine compound | Heat resistance evaluation results |
|---|---|---|
| Example 1 | LA-F70 | A |
| Example 2 | TINUVIN 460 | B |
| Comparative Example 1 | CR-1 | C |

[Examples 3 to 5 and Comparative Example 2]

(Preparation of Resist Composition)

A resist composition was prepared according to the recipe shown in Table 2 using the film forming materials. Among the components of the resist composition in Table 2, the following acid generating agent (C), acid diffusion controlling agent (E), and solvent were used.

Acid generating agent (C): P-1: triphenylsulfonium trifluoromethanesulfonate (Midori Kagaku Co., Ltd.)
Acid diffusion controlling agent(E): Q-1: trioctylamine (Tokyo Kasei Kogyo Co., Ltd.)
Solvent: S-1: propylene glycol monomethyl ether (Tokyo Kasei Kogyo Co., Ltd.)

(Method for evaluating resist performance of resist composition)

A clean silicon wafer was spin coated with the homogeneous resist composition, and then prebaked (PB) before exposure in an oven of 110°C to form a resist film with a thickness of 60 nm. The obtained resist film was irradiated with electron beams of 1:1 line and space setting with a 80 nm interval using an electron beam lithography system (ELS-7500 manufactured by ELIONIX INC.). After irradiation, the resist film was heated at each predetermined temperature for 90 seconds, and immersed in 2.38% by mass TMAH alkaline developer for 60 seconds for development. Subsequently, the resist film was washed with ultrapure water for 30 seconds, and dried to form a positive type resist pattern. Concerning the formed resist pattern, the line and space were observed by a scanning electron microscope (S-4800 manufactured by Hitachi High-Technologies Corporation) to evaluate the reactivity by electron beam irradiation of the resist composition.

[Table 2]

| | Film forming material | Resist composition | | | | Resist performance evaluation |
|---|---|---|---|---|---|---|
| | | Film forming material [g] | P-1 [g] | Q-1 [g] | S-1 [g] | |
| Example 3 | LA-F70 | 1.0 | 0.3 | 0.03 | 100.0 | Good |
| Example 4 | TINUVIN 460 | 1.0 | 0.3 | 0.03 | 100.0 | Good |
| Example 5 | LA-F70 | 1.0 | 0.0 | 0.0 | 100.0 | Good |
| Comparative Example 2 | CR-1 | 1.0 | 0.3 | 0.03 | 100.0 | Poor |

As is evident from Table 2, in resist pattern evaluation, a good resist pattern was able to be obtained by irradiation with electron beams of 1:1 line and space setting with an 80 nm interval in Examples 3 to 5. On the other hand, no good resist pattern was able to be obtained in Comparative Example 2.

As described above, the compound that satisfies the requirements of the present invention has high heat resistance and can impart a good shape to a resist pattern, as compared with the comparative compound (CR-1). As long as the requirements of the present invention are met, compounds other than those described in Examples also exhibit the same effects.

[Examples 6 to 8]

(Preparation of composition for underlayer film formation for lithography)

Compositions for underlayer film formation for lithography were prepared according to the composition shown in the following Table 3. Among the components of the compositions for underlayer film formation for lithography in Table 3, the following acid generating agent, crosslinking agent, and solvent were used. Acid generating agent: di-tertiary butyl diphenyliodonium nonafluoromethanesulfonate (DTDPI) manufactured by Midori Kagaku Co., Ltd.

Crosslinking agent: NIKALAC MX270 (NIKALAC) (Sanwa Chemical Co., Ltd.)
Solvent: propylene glycol monomethyl ether acetate (PGMEA)

[Table 3]

| | Underlayer film forming material (parts by mass) | Solvent (parts by mass) | Acid generating agent (parts by mass) | Crosslinking agent (parts by mass) |
|---|---|---|---|---|
| Example 6 | LA-F70 (1) | PGMEA (90) | DTDPI (0.5) | Nikalac (0.5) |
| Example 7 | TINUVIN460 (1) | PGMEA (90) | DTDPI (0.5) | Nikalac (0.5) |
| Example 8 | LA-F70 (1) | PGMEA (90) | DTDPI (0.0) | Nikalac (0.0) |

[Example 9]

A $SiO_2$ substrate with a film thickness of 300 nm was coated with the composition for underlayer film formation for lithography of Example 6, and baked at 240°C for 60 seconds and further at 400°C for 120 seconds to form an underlayer film with a film thickness of 85 nm. This underlayer film was coated with a resist solution for ArF and baked at 130°C for 60 seconds to form a photoresist layer with a film thickness of 140 nm.

The ArF resist solution used was prepared by containing 5 parts by mass of a compound of the following formula (16), 1 part by mass of triphenylsulfonium nonafluoromethanesulfonate, 2 parts by mass of tributylamine, and 92 parts by mass of PGMEA.

The compound of the formula (16) was prepared as follows.

4.15 g of 2-methyl-2-methacryloyloxyadamantane, 3.00 g of methacryloyloxy-γ-butyrolactone, 2.08 g of 3-hydroxy-1-adamantyl methacrylate, and 0.38 g of azobisisobutyronitrile were dissolved in 80 mL of tetrahydrofuran to prepare a reaction solution. This reaction solution was polymerized for 22 hours with the reaction temperature kept at 63°C in a nitrogen atmosphere. Then, the reaction solution was added dropwise into 400 mL of n-hexane. The product resin thus obtained was solidified and purified, and the resulting white powder was filtered and dried overnight at 40°C under reduced pressure to obtain a compound represented by the following formula (16).

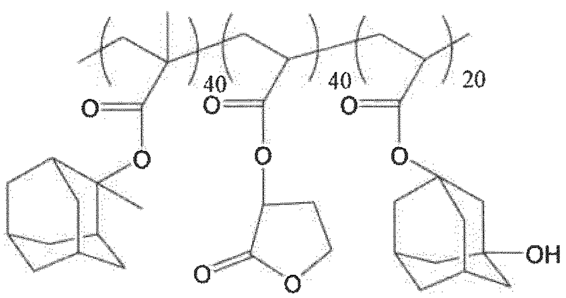

(16)

wherein 40, 40, and 20 represent the ratio of each constituent unit and do not represent a block copolymer.

Subsequently, the photoresist layer was exposed using an electron beam lithography system (manufactured by ELIONIX INC.; ELS-7500, 50 keV), baked (PEB) at 115°C for 90 seconds, and developed for 60 seconds in 2.38%

by mass tetramethylammonium hydroxide (TMAH) aqueous solution to obtain a positive type resist pattern. The evaluation results are shown in Table 4.

[Example 10]

A positive type resist pattern was obtained in the same manner as in Example 9, except that the composition for underlayer film formation for lithography in Example 7 was used instead of the composition for underlayer film formation for lithography in Example 6. The evaluation results are shown in Table 4.

[Example 11]

A positive type resist pattern was obtained in the same manner as in Example 9, except that the composition for underlayer film formation for lithography in Example 8 was used instead of the composition for underlayer film formation for lithography in Example 6. The evaluation results are shown in Table 4.

[Comparative Example 3]

The same operations as in Example 10 were performed except that no underlayer film was formed so that a photoresist layer was formed directly on a $SiO_2$ substrate to obtain a positive type resist pattern. The evaluation results are shown in Table 4.

[Evaluation]

Concerning each of Examples 9 to 11 and Comparative Example 3, the shapes of the obtained 55 nm L/S (1:1) and 80 nm L/S (1:1) resist patterns were observed under an electron microscope manufactured by Hitachi, Ltd. (S-4800). The shapes of the resist patterns after development were evaluated as "goodness" when having good rectangularity without pattern collapse, and as "poorness" if this was not the case. The smallest line width having good rectangularity without pattern collapse as a result of this observation was used as an index for evaluating the resolution. The smallest electron beam energy quantity capable of lithographing good pattern shapes was used as an index for sensitivity evaluation.

[Table 4]

| | Composition for film formation for lithography | Resolution (nmL/S) | Sensitivity (μC/cm2) | Resist pattern shape after development |
|---|---|---|---|---|
| Example 9 | Composition of Example 6 | 56 | 17 | Good |
| Example 10 | Composition of Example 7 | 60 | 18 | Good |
| Example 11 | Composition of Example 8 | 64 | 20 | Good |
| Comparative Example 3 | None | 90 | 42 | Poor |

As is evident from the results shown in Table 4, it has been confirmed that Examples 9 to 11 using the composition for film formation for lithography of the present embodiment containing a triazine-based compound were significantly superior in both resolution and sensitivity as compared with Comparative Example 3. Also, the resist pattern shapes after development were confirmed to have good rectangularity without pattern collapse. The difference in the resist pattern shapes after development indicated that the underlayer film obtained from the composition for film formation for lithography of Examples 9 to 11 has good adhesiveness to a resist material.

[Examples 12 to 14]

A composition for optical component formation was prepared with the recipe shown in Table 5 below. Among the compositions for optical component formation in Table 5, the following acid generating agent, acid crosslinking agent, acid diffusion controlling agent, and solvent were used.

- Acid generating agent: di-tertiary butyl diphenyliodonium nonafluoromethanesulfonate (DTDPI) manufactured by Midori Kagaku Co. Ltd.

- Crosslinking agent: NIKALAC MX270 (NIKALAC) (Sanwa Chemical Co. Ltd.)
- Organic solvent: propylene glycol monomethyl ether acetate (PGMEA)

A clean silicon wafer was spin coated with a composition for optical component formation in a homogeneous state, and then prebaked (PB) in an oven of 110°C to form an optical component forming-film with a thickness of 1 μm. The prepared composition for optical component formation was evaluated as "A" in a case where the film formation was good and "C" in a case where the formed film had defects.

A clean silicon wafer was spin coated with a homogeneous composition for optical component formation, and then PB was performed in an oven of 110°C to form a film with a thickness of 1 μm. For the formed film, the refractive index (λ = 589.3 nm) at 25°C was measured with a multiple incident angle spectro ellipsometer VASE manufactured by JA Woollam, and in a case where the refractive index was 1.6 or more, the formed films were evaluated as "A", "B" in a case of 1.55 or more and less than 1.6, and "C" in a case of less than 1.55. In a case where the transmittance (λ = 632.8 nm) was 90% or more, the formed film was evaluated as "A" was evaluated, and evaluated as "C" when the transmittance was evaluated less than 90%.

[Table 5]

| | Underlayer film forming material (parts by mass) | Solvent (parts by mass) | Acid generating agent (parts by mass) | Crosslinking agent (parts by mass) | Film formation evaluation | Refractive index evaluation | Transmittance evaluation |
|---|---|---|---|---|---|---|---|
| Example 12 | LA-F70 (10) | PGMEA (190) | DTDPI (0.5) | Nikalac (2.0) | A | A | A |
| Example 13 | TINUVIN460 (10) | PGMEA (190) | DTDPI (0.5) | Nikalac (2.0) | A | A | A |
| Example 14 | LA-F70 (10) | PGMEA (190) | DTDPI (0.0) | Nikalac (0.0) | A | A | A |
| Comparative Example 4 | CR-1 (10) | PGMEA (190) | DTDPI (0.5) | Nikalac (2.0) | A | C | C |

<Synthesis Working Example 1> Synthesis of BisN-20

A four necked flask (internal capacity: 1 L) equipped with a Dimroth condenser tube, a thermometer, and a stirring blade and having a detachable bottom was prepared. A solution obtained by adding 12.0 g (17.1 mmol) of a triazine compound (LA-F70 manufactured by ADEKA Co., Ltd.) and 6.2 g (45 mmol) of potassium carbonate to 100 mL acetone in a nitrogen stream was added to this four necked flask, and further, 3.24 g (45 mmol) of acrylic acid was added, and the resulting reaction solution was stirred for 7 hours under reflux to carry out the reaction. Next, the solid component was removed from the reaction solution by filtration, cooled in an ice bath, and the reaction solution was concentrated to precipitate a solid. The precipitated solid matter was filtrated and dried and then separated and purified by column chromatography to obtain 4.1 g of the objective compound represented by the following formula (BisN-20).

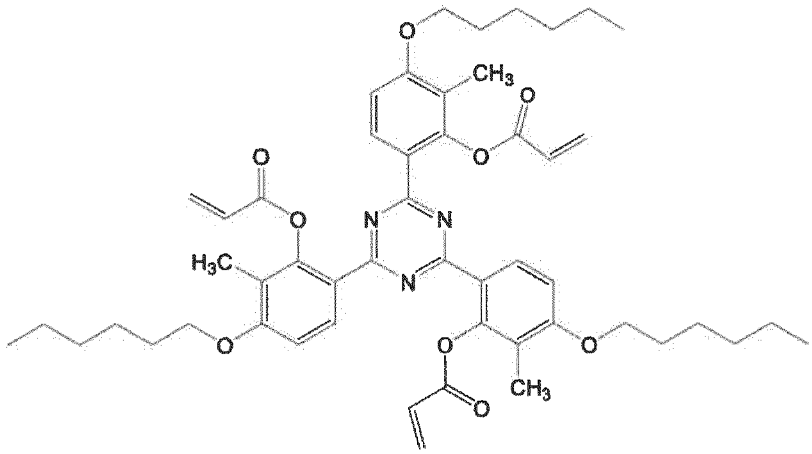

ＢｉｓＮ－２０

<Synthesis Working Example 2> Synthesis of BisN-21

A four necked flask (internal capacity: 0.5 L) equipped with a Dimroth condenser tube, a thermometer, and a stirring blade was prepared. A solution obtained by adding 12.0 g (17.1 mmol) of a triazine compound (LA-F70 manufactured by ADEKA Co., Ltd.) and 6.2 g (45 mmol) of potassium carbonate to 100 mL acetone under a nitrogen stream was added to this four necked flask, and further, 3.87 g (45 mmol) of methacrylic acid was added, and the resulting reaction solution was stirred for 7 hours under reflux to carry out the reaction. Next, the solid component was removed from the reaction solution by filtration, cooled in an ice bath, and the reaction solution was concentrated to precipitate a solid. The solid matter obtained by filtration was dried and then separated and purified by column chromatography to obtain 3.9 g of the objective compound represented by the following formula (BisN-21).

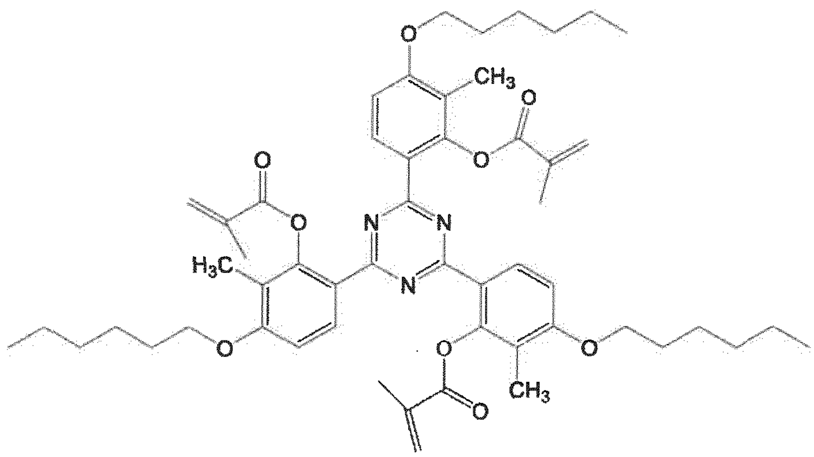

ＢｉｓＮ－２１

<Synthesis Working Example 3> Synthesis of BisN-22

A four necked flask (internal capacity: 0.5 L) equipped with a Dimroth condenser tube, a thermometer, and a stirring blade was prepared. A solution obtained by adding 12.0 g (17.1 mmol) of a triazine compound (LA-F70 manufactured by ADEKA Co., Ltd.) and 6.2 g (45 mmol) of potassium carbonate to 100 mL dimethylacetamide under a nitrogen stream was added to this four necked flask, and further, 4.2g (45 mmol) of epichlorohydrin was added, and the resulting reaction solution was stirred 90°C for 6.5 hours to carry out the reaction. Next, the solid component was removed from the reaction solution by filtration, cooled in an ice bath, and the reaction solution was concentrated to precipitate a solid. The precipitated solid matter was filtrated and dried and then separated and purified by column chromatography to obtain 4.0 g of the objective compound represented by the following formula (BisN-22).

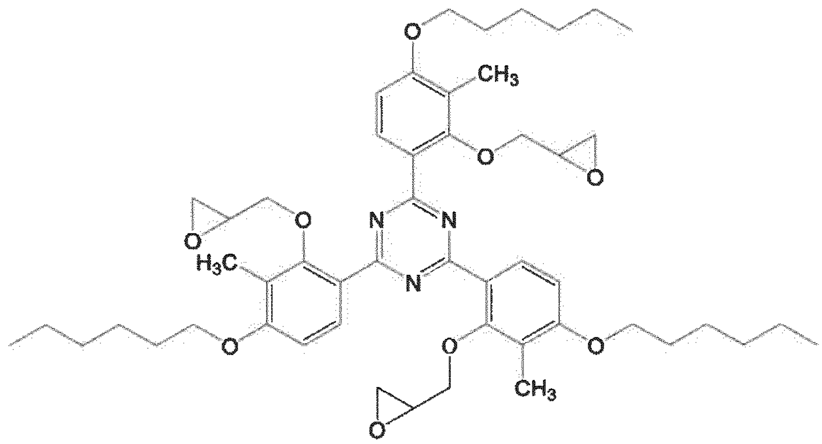

BisN-22

<Synthesis Working Example 4> Synthesis of BisN-23

A four necked flask (internal capacity: 0.5 L) equipped with a Dimroth condenser tube, a thermometer, and a stirring blade was prepared. A solution obtained by adding 12.0 g (17.1 mmol) of a triazine compound (LA-F70 manufactured by ADEKA Co., Ltd.) and 6.2 g (45 mmol) of potassium carbonate to 100 mL acetone under a nitrogen stream was added to this four necked flask, and further, 5.4 g (45 mmol) of allyl bromide and 2.0 g of 18-crown-6, was added, and the resulting reaction solution was stirred for 6.5 hours under reflux to carry out the reaction. Next, the solid component was removed from the reaction solution by filtration, cooled in an ice bath, and the reaction solution was concentrated to precipitate a solid. The solid matter obtained by filtration was dried and then separated and purified by column chromatography to obtain 4.0 g of the objective compound represented by the following formula (BisN-23).

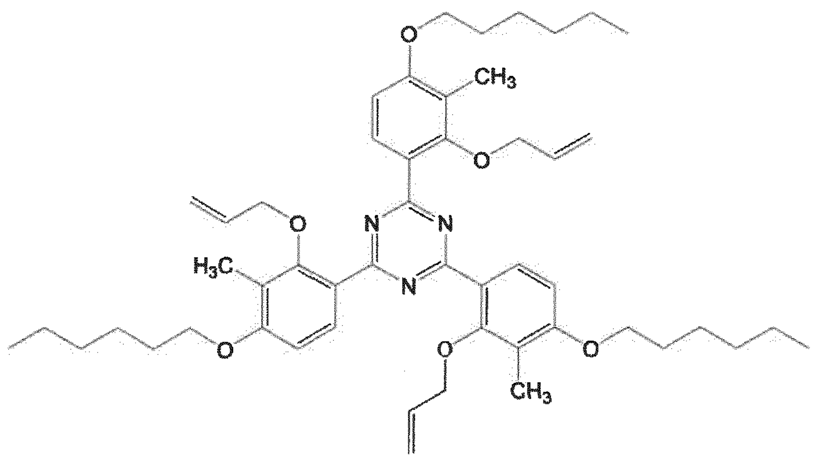

BisN-23

<Synthesis Working Example 5> Synthesis of BisN-24

A four necked flask (internal capacity: 1 L) equipped with a Dimroth condenser tube, a thermometer, and a stirring blade and having a detachable bottom was prepared. A solution obtained by adding 12.0 g (19.0 mmol) of a triazine compound (TINUVIN 460 manufactured by BASF Corporation) and 6.2 g (45 mmol) of potassium carbonate to 100 mL acetone in a nitrogen stream was added to this four necked flask, and further, 3.24 g (45 mmol) of acrylic acid was added, and the resulting reaction solution was stirred for 7 hours under reflux to carry out the reaction. Next, the solid component was removed from the reaction solution by filtration, cooled in an ice bath, and the reaction solution was concentrated to precipitate a solid. The solid matter obtained by filtration was dried and then separated and purified by column chromatography to obtain 4.2 g of the objective compound represented by the following formula (BisN-24).

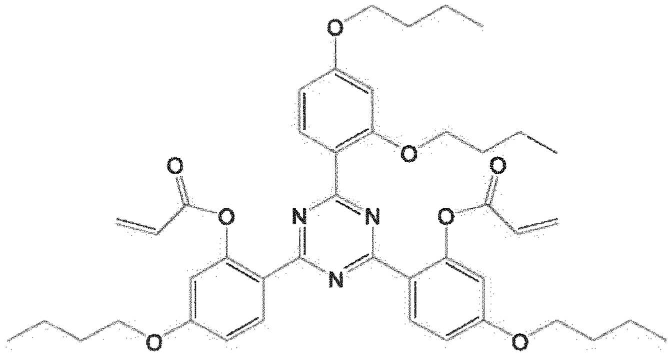

BisN-24

(In the BisN-24, each of the three benzene rings directly bonded to the triazine ring can rotate.)

<Synthesis Working Example 6> Synthesis of BisN-25

A four necked flask (internal capacity: 0.5 L) equipped with a Dimroth condenser tube, a thermometer, and a stirring blade was prepared. A solution obtained by adding 12.0 g (19.0 mmol) of a triazine compound (TINUVIN 460 manufactured by BASF Corporation) and 6.2 g (45 mmol) of potassium carbonate to 100 mL acetone under a nitrogen stream was added to this four necked flask, and further, 3.87 g (45 mmol) of methacrylic acid was added, and the resulting reaction solution was stirred for 7 hours under reflux to carry out the reaction. Next, the solid component was removed from the reaction solution by filtration, cooled in an ice bath, and the reaction solution was concentrated to precipitate a solid. The solid matter obtained by filtration was dried and then separated and purified by column chromatography to obtain 3.9 g of the objective compound (BisN-25) represented by the following formula.

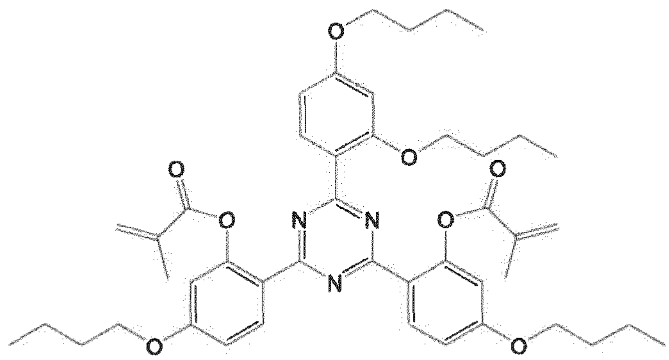

BisN-25

(In the BisN-25, each of the three benzene rings directly bonded to the triazine ring can rotate.)

<Synthesis Working Example 7> Synthesis of BisN-26

A four necked flask (internal capacity: 0.5 L) equipped with a Dimroth condenser tube, a thermometer, and a stirring blade was prepared. A solution obtained by adding 12.0 g (19.0 mmol) of a triazine compound (TINUVIN 460

manufactured by BASF Corporation) and 6.2 g (45 mmol) of potassium carbonate to 100 mL dimethylacetamide under a nitrogen stream was added to this four necked flask, and further, 4.2g (45 mmol) of epichlorohydrin was added, and the resulting reaction solution was stirred 90°C for 6.5 hours to carry out the reaction. Next, the solid component was removed from the reaction solution by filtration, cooled in an ice bath, and the reaction solution was concentrated to precipitate a solid. The solid matter obtained by filtration was dried and then separated and purified by column chromatography to obtain 4.03 g of the objective compound (BisN-26) represented by the following formula.

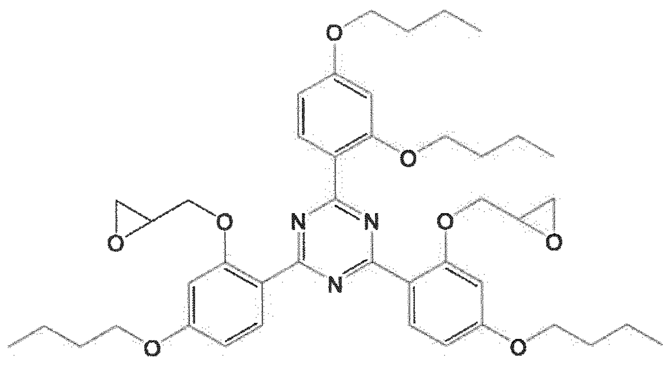

BisN-26

(In the BisN-26, each of the three benzene rings directly bonded to the triazine ring can rotate.)

<Synthesis Working Example 8> Synthesis of BisN-27

A four necked flask (internal capacity: 0.5 L) equipped with a Dimroth condenser tube, a thermometer, and a stirring blade was prepared. A solution obtained by adding 12.0 g (19.0 mmol) of a triazine compound (TINUVIN 460 manufactured by BASF Corporation) and 6.2 g (45 mmol) of potassium carbonate to 100 mL acetone under a nitrogen stream was added to this four necked flask, and further, 5.4 g (45 mmol) of allyl bromide and 2.0 g of 18-crown-6, was added, and the resulting reaction solution was stirred for 6.5 hours under reflux to carry out the reaction. Next, the solid component was removed from the reaction solution by filtration, cooled in an ice bath, and the reaction solution was concentrated to precipitate a solid. The solid matter obtained by filtration was dried and then separated and purified by column chromatography to obtain 4.0 g of the objective compound (BisN-27) represented by the following formula.

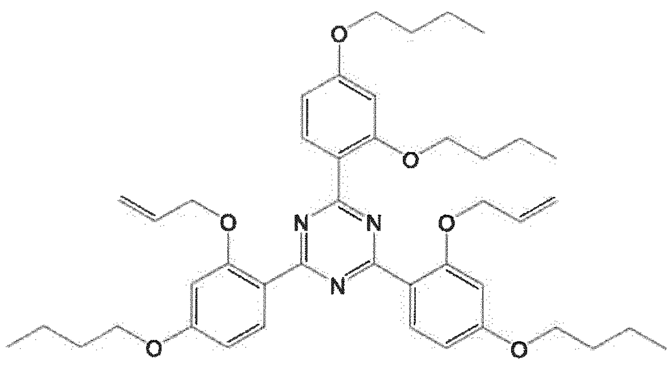

BisN-27

(In the BisN-27, each of the three benzene rings directly bonded to the triazine ring can rotate.)

[Method for evaluating UV irradiation curability (Examples 15 to 22 and Comparative Examples 5 to 6)]

The compositions for film formation for lithography of each of Examples 15 to 30 and Comparative Examples 5 to 6 prepared with compositions shown in Table 6 (in the table, "content" indicates % by mass) were spin coated on a clean silicon wafer and baked in an oven at 240°C for 60 seconds to form a baked film. The baked film was irradiated with UV at a wavelength of 360 nm using a UV irradiation apparatus (BJB 267: high pressure mercury lamp, manufactured by GS Yuasa Co., Ltd.) to obtain a UV cured film. After measuring the film thickness of the UV cured film with an ellipsometer (manufactured by FiveLab Co., Ltd., laser wavelength: 632.8 nm), the UV cured film was immersed in

propylene glycol monomethyl ether acetate (PGMEA) for 60 seconds at room temperature. Thereafter, air was blown to the film and further heated at 100°C for 60 seconds to remove the solvent. Thereafter, the thickness of the UV cured film was measured again with an ellipsometer, and the film remaining rate after immersion in solvent was calculated according to the following equation.

Film remaining rate (%) = UV cured film thickness after solvent immersion / UV cured film thickness before solvent immersion × 100

The UV curability was evaluated from the calculated film remaining rate according to the following criteria. The evaluation results are shown in Table 6. From the practical viewpoint, for application to the UV curing semiconductor microfabrication process, "S" is most preferred, followed by "A", followed by "B", and it was evaluated that up to "C" was a level could be applied to the UV curing process. The "D" was evaluated as not applicable to the UV curing process.

<Evaluation criteria>

S: The film remaining rate was 90% or more.
A: The film remaining rate was 80% or more and less than 90%.
B: The film remaining rate was 50% or more and less than 80%.
C: The film remaining rate was 20% or more and less than 50%.
D: The film remaining rate was less than 20%.

[Table 6]

| | Composition for film formation for lithography | | | | | | Measurement conditions and results | | | |
|---|---|---|---|---|---|---|---|---|---|---|
| | Resin | | Solvent | | Photopolymerization initiator | | Film remaining rate | UV irradiation | Immersion solvent | UV curability evaluation |
| | Type | Content | Type | Content | Type | Content | (%) | (mj/gcm2) | | |
| Example 15 | BisN-20 | 10 | MEK | 85 | IRGACURE651 | 5 | 97.6 | 1500 | PGMEA | S |
| Example 16 | BisN-21 | 10 | MEK | 85 | IRGACURE651 | 5 | 98.8 | 1500 | PGMEA | S |
| Example 17 | BisN-22 | 10 | MEK | 85 | IRGACURE651 | 5 | 98.6 | 1500 | PGMEA | S |
| Example 18 | BisN-23 | 10 | MEK | 85 | IRGACURE651 | 5 | 98.1 | 1500 | PGMEA | S |
| Example 19 | BisN-20 | 10 | MEK | 90 | - | - | 87.2 | 1500 | PGMEA | A |
| Example 20 | BisN-21 | 10 | MEK | 90 | - | - | 88.9 | 1500 | PGMEA | A |
| Example 21 | BisN-22 | 10 | MEK | 90 | - | - | 87.1 | 1500 | PGMEA | A |
| Example 22 | BisN-23 | 10 | MEK | 90 | - | - | 87.4 | 1500 | PGMEA | A |
| Example 23 | BisN-24 | 10 | MEK | 85 | IRGACURE651 | 5 | 68.7 | 1500 | PGMEA | B |
| Example 24 | BisN-25 | 10 | MEK | 85 | IRGACURE651 | 5 | 70.2 | 1500 | PGMEA | B |
| Example 25 | BisN-26 | 10 | MEK | 85 | IRGACURE651 | 5 | 69.2. | 1500 | PGMEA | B |
| Example 26 | BisN-27 | 10 | MEK | 85 | IRGACURE651 | 5 | 69.2 | 1500 | PGMEA | B |
| Example 27 | BisN-24 | 10 | MEK | 90 | - | - | 49.4 | 1500 | PGMEA | C |

(continued)

| | Composition for film formation for lithography | | | | | | Measurement conditions and results | | | |
|---|---|---|---|---|---|---|---|---|---|---|
| | Resin | | Solvent | | Photopolymerization initiator | | Film remaining rate | UV irradiation | Immersion solvent | UV curability evaluation |
| | Type | Content | Type | Content | Type | Content | (%) | (mj/gcm2) | | |
| Example 28 | BisN-25 | 10 | MEK | 90 | - | - | 51.8 | 1500 | PGMEA | B |
| Example 29 | BisN-26 | 10 | MEK | 90 | - | - | 49.3 | 1500 | PGMEA | C |
| Example 30 | BisN-27 | 10 | MEK | 90 | - | - | 49.9 | 1500 | PGMEA | C |
| Comparative Example 5 | CR-1 | 10 | MEK | 85 | IRGACURE651 | 5 | 0 | 1500 | PGMEA | D |
| Comparative Example 6 | CR-1 | 10 | MEK | 90 | - | - | 0 | 1500 | PGMEA | D |

As shown in Table 6, Examples 15 to 30 exhibited good UV curing properties. In particular, it was confirmed that the UV curing characteristics can be improved when the photopolymerization initiator is contained. On the other hand, in Comparative Examples 5 and 6, UV curing was not performed.

This application claims a priority from a Japanese patent application (Japanese Patent Application No. 2017-126543) filed on June 28, 2017, the contents of which are incorporated herein by reference.

Industrial Applicability

The material for film formation of the present embodiment has relatively high heat resistance and also relatively high solvent solubility and are applicable to a wet process. Therefore, the composition for film formation for lithography containing the film forming material of the present embodiment can be used widely and effectively for various purposes required to have such performance. In particular, the present invention can be particularly effectively used in the fields of underlayer films for lithography and underlayer films for multilayer resists. Furthermore, since the material for film formation of the present embodiment is excellent in heat resistance, transparency and refractive index, it is possible to effectively use particularly as a material for optical component formation.

## Claims

**1.** A film forming material comprising a triazine-based compound represented by the following formula (1):

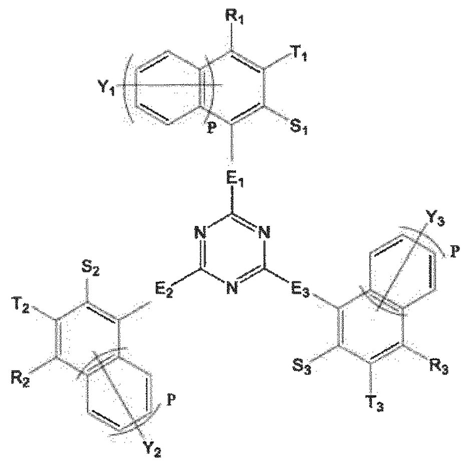

(1)

wherein $R_1$, $R_2$, and $R_3$ each independently represent a hydrogen atom, a linear alkyl group having 1 to 30 carbon atoms and optionally having a substituent, a branched alkyl group having 1 to 30 carbon atoms and optionally having a substituent, a cycloalkyl group having 3 to 30 carbon atoms and optionally having a substituent, an aryl group having 6 to 30 carbon atoms and optionally having a substituent, an alkenyl group having 2 to 30 carbon atoms and optionally having a substituent, an alkoxy group having 1 to 30 carbon atoms and optionally having a substituent, an alkylaryl group having 7 to 30 carbon atoms and optionally having a substituent, an arylalkyl group having 7 to 30 carbon atoms and optionally having a substituent, a hydroxyl group, or a group in which a hydrogen atom of a hydroxyl group is replaced with an acid dissociation group or a crosslinkable reactive group, wherein the alkyl group, the cycloalkyl group, the aryl group, the alkenyl group, the alkoxy group, the alkylaryl group, or the arylalkyl group each optionally comprises an ether bond, a ketone bond, an ester bond, or a crosslinkable reactive group; $S_1$, $S_2$, and $S_3$ each independently represent a hydrogen atom, a hydroxyl group, a group in which a hydrogen atom of a hydroxyl group is replaced with an acid dissociation group or a crosslinkable reactive group, or an alkoxy group having 1 to 30 carbon atoms; $T_1$, $T_2$, and $T_3$ each independently represent a hydrogen atom, a hydroxyl group, an alkyl group having 1 to 30 carbon atoms, or an alkenyl group having 2 to 30 carbon atoms; $Y_1$, $Y_2$, and $Y_3$ each independently represent a hydrogen atom, a hydroxyl group, a group in which a hydrogen atom of a hydroxyl group is replaced with an acid dissociation group or a crosslinkable reactive group, an alkyl, alkoxy, alkoxycarbonyl or arylalkyl group having 1 to 30 carbon atoms, or an alkenyl group having 2 to 30 carbon atoms; $E_1$, $E_2$, and $E_3$ each independently represent a single bond, -O-, $-CH_2O-$, -COO-, or -NH-; and each P independently represents an integer of 0 to 1.

**2.** The film forming material according to claim 1, wherein the triazine-based compound represented by the formula (1) is a triazine-based compound represented by the following formula (2):

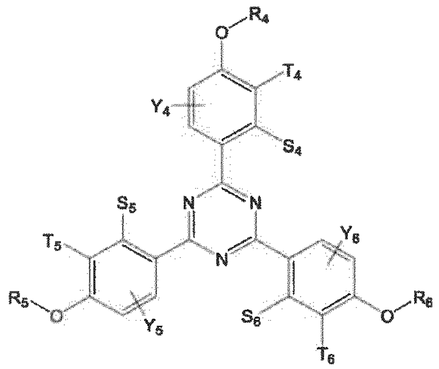

(2)

wherein $R_4$, $R_5$, and $R_6$ each independently represent a linear or branched alkyl group having 1 to 12 carbon atoms, a cycloalkyl group having 3 to 8 carbon atoms, an alkenyl group having 3 to 8 carbon atoms, an aryl group having 6 to 18 carbon atoms, or an alkylaryl or arylalkyl group having 7 to 18 carbon atoms, wherein the alkyl group, the cycloalkyl group, the alkenyl group, the aryl group, the alkylaryl group or the arylalkyl group is each optionally substituted with a hydroxyl group, or an alkyl or alkoxy group having 1 to 12 carbon atoms, wherein the alkyl group, the cycloalkyl group, the alkenyl group, the aryl group, the alkylaryl group, or the arylalkyl group each optionally comprises an ether bond, a ketone bond, or an ester bond; $S_4$, $S_5$, and $S_6$ each independently represent a hydrogen atom, a hydroxyl group, a group in which a hydrogen atom of a hydroxyl group is replaced with an acid dissociation group or a crosslinkable reactive group, or an alkoxy group having 1 to 4 carbon atoms; $T_4$, $T_5$, and $T_6$ each independently represent a hydrogen atom, a hydroxyl group, an alkyl group having 1 to 8 carbon atoms, or an alkenyl group having 2 to 8 carbon atoms; and $Y_4$, $Y_5$, and $Y_6$ each independently represent a hydrogen atom, a hydroxyl group, a group in which a hydrogen atom of a hydroxyl group is replaced with an acid dissociation group or a crosslinkable reactive group, an alkyl, alkoxy, alkoxycarbonyl or arylalkyl group having 1 to 12 carbon atoms, or an alkenyl group having 2 to 8 carbon atoms.

**3.** The film forming material according to claim 2, wherein the triazine-based compound represented by the formula (2) is a triazine-based compound represented by the following formula (3):

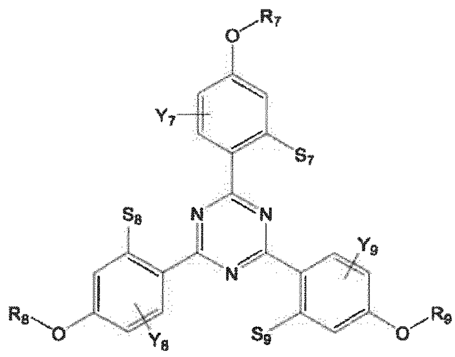

(3)

wherein $R_7$, $R_8$, and $R_9$ each independently represent a linear or branched alkyl group having 1 to 12 carbon atoms, a cycloalkyl group having 3 to 8 carbon atoms, or an alkenyl group having 3 to 8 carbon atoms, an aryl group having 6 to 18 carbon atoms, or an alkylaryl or arylalkyl group having 7 to 18 carbon atoms, wherein the alkyl group, the cycloalkyl group, the alkenyl group, the aryl group, the alkylaryl group or the arylalkyl group is each optionally substituted with a hydroxyl group, or an alkyl or alkoxy group having 1 to 12 carbon atoms, and wherein the alkyl group, the cycloalkyl group, the alkenyl group, the aryl group, the alkylaryl group, or the arylalkyl group each optionally comprises an ether bond, a ketone bond, or an ester bond; $S_7$, $S_8$, and $S_9$ each independently represent a hydrogen atom, a hydroxyl group, a group in which a hydrogen atom of a hydroxyl group is replaced with an acid dissociation group or a crosslinkable reactive group, or an alkoxy group having 1 to 4 carbon atoms; and $Y_7$, $Y_8$, and $Y_9$ each

independently represent a hydrogen atom, a hydroxyl group, a group in which a hydrogen atom of a hydroxyl group is replaced with an acid dissociation group or a crosslinkable reactive group, an alkyl, alkoxy, alkoxycarbonyl or arylalkyl group having 1 to 12 carbon atoms, or an alkenyl group having 2 to 8 carbon atoms.

**4.** The film forming material according to claim 3, wherein the triazine-based compound represented by the formula (3) is a triazine-based compound represented by the following formula (4):

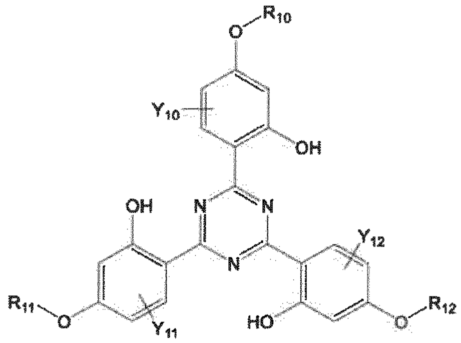

(4)

wherein $R_{10}$, $R_{11}$, and $R_{12}$ each independently represent a linear or branched alkyl group having 1 to 12 carbon atoms, a cycloalkyl group having 3 to 8 carbon atoms, or an alkenyl group having 3 to 8 carbon atoms, an aryl group having 6 to 18 carbon atoms, or an alkylaryl or arylalkyl group having 7 to 18 carbon atoms, wherein the alkyl group, the cycloalkyl group, the alkenyl group, the aryl group, the alkylaryl group or the arylalkyl group is each optionally substituted with a hydroxyl group, or an alkyl or alkoxy group having 1 to 12 carbon atoms, and wherein the alkyl group, the cycloalkyl group, the alkenyl group, the aryl group, the alkylaryl group, or the arylalkyl group each optionally comprises an ether bond, a ketone bond, or an ester bond; and $Y_{10}$, $Y_{11}$, and $Y_{12}$ each independently represent a hydrogen atom, a hydroxyl group, a group in which a hydrogen atom of a hydroxyl group is replaced with an acid dissociation group or a crosslinkable reactive group, an alkyl, alkoxy group, alkoxycarbonyl or arylalkyl group having 1 to 12 carbon atoms, or an alkenyl group having 2 to 8 carbon atoms.

**5.** The film forming material according to claim 2, wherein the triazine-based compound represented by the formula (2) is a triazine-based compound represented by the following formula (5) :

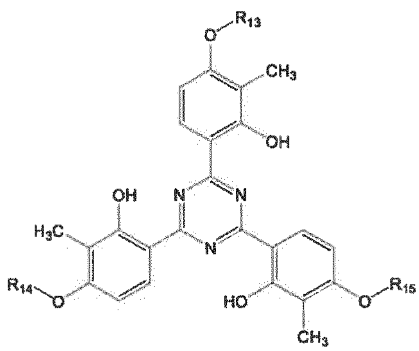

(5)

wherein $R_{13}$, $R_{14}$, and $R_{15}$ each independently represent a linear or branched alkyl group having 1 to 12 carbon atoms, wherein the alkyl group is optionally substituted with a hydroxyl group or an alkoxy group having 1 to 12 carbon atoms, and wherein the alkyl group optionally comprises an ether bond, a ketone bond or an ester bond.

**6.** The film forming material according to claim 5, wherein the triazine-based compound represented by the formula (5) is a triazine-based compound represented by the following formula (BisN-8):

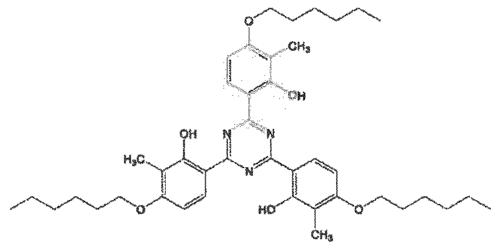

(B i s N − 8)

7. A film forming material comprising a triazine-based compound represented by the following formula (6):

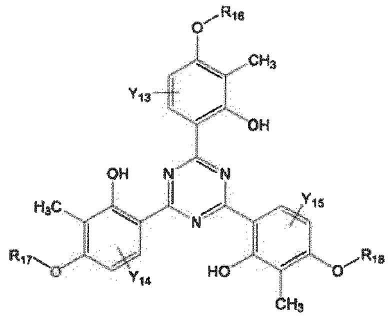

(6)

wherein $R_{16}$, $R_{17}$, and $R_{18}$ each independently represent a linear or branched alkyl group having 1 to 12 carbon atoms and substituted with a methacryloyloxy group or an acryloyloxy group, wherein the alkyl group is optionally substituted with a hydroxyl group, an alkoxy group having 1 to 8 carbon atoms, or an acyloxy group having 1 to 8 carbon atoms, and wherein the alkyl group optionally comprises an ether bond, a ketone bond or an ester bond; and $Y_{13}$, $Y_{14}$, and $Y_{15}$ each independently represent a hydrogen atom, a hydroxyl group, a group in which a hydrogen atom of a hydroxyl group is replaced with an acid dissociation group or a crosslinkable reactive group, an alkyl, alkoxy, alkoxycarbonyl or arylalkyl group having 1 to 12 carbon atoms, or an alkenyl group having 2 to 8 carbon atoms.

8. The film forming material according to claim 7, further comprising:

one or more selected from the group consisting of a photocurable monomer, a photocurable oligomer, and a photocurable polymer, and
a photopolymerization initiator.

9. A composition for film formation for lithography comprising one or more selected from the group consisting of the film forming material according to any one of claims 1 to 8.

10. A material for optical component formation comprising one or more selected from the group consisting of the film forming material according to any one of claims 1 to 8.

11. A resist composition comprising one or more selected from the group consisting of the film forming material according to any one of claims 1 to 8.

12. The resist composition according to claim 11, further comprising a solvent.

13. The resist composition according to claim 11 or 12, further comprising an acid generating agent.

14. The resist composition according to any one of claims 11 to 13, further comprising an acid diffusion controlling agent.

15. A method for forming a resist pattern, comprising:

forming a resist film on a substrate using the resist composition according to any one of claims 11 to 14;
exposing at least a portion of the resist film; and

developing the exposed resist film, thereby forming a resist pattern.

**16.** A permanent film for a resist obtained from the resist composition according to any one of claims 11 to 14.

**17.** A radiation-sensitive composition comprising:

a component (A) which is one or more selected from the group consisting of the film forming material according to any one of claims 1 to 8,
an optically active diazonaphthoquinone compound (B), and
a solvent,
wherein a content of the solvent is 20 to 99% by mass based on 100% by mass in total of the radiation-sensitive composition.

**18.** The radiation-sensitive composition according to claim 17, wherein a content ratio among the component (A), the optically active diazonaphthoquinone compound (B), and a further optional component (D) ((A)/(B)/(D)) is 1 to 99% by mass/99 to 1% by mass/0 to 98% by mass based on 100% by mass of solid components of the radiation-sensitive composition.

**19.** The radiation-sensitive composition according to claim 17 or 18, wherein the radiation-sensitive composition is capable of forming an amorphous film by spin coating.

**20.** A method for producing an amorphous film, comprising forming an amorphous film on a substrate using the radiation-sensitive composition according to any one of claims 17 to 19.

**21.** A method for forming a resist pattern, comprising:

forming a resist film on a substrate using the radiation-sensitive composition according to any one of claims 17 to 19;
exposing at least a portion of the resist film; and
developing the exposed resist film, thereby forming a resist pattern.

**22.** An underlayer film forming material for lithography comprising one or more selected from the group consisting of the film forming material according to any one of claims 1 to 8.

**23.** A composition for underlayer film formation for lithography comprising the underlayer film forming material for lithography according to claim 22, and a solvent.

**24.** The composition for underlayer film formation for lithography according to claim 23, further comprising an acid generating agent.

**25.** The composition for underlayer film formation for lithography according to claim 23 or 24, further comprising a crosslinking agent.

**26.** A method for producing an underlayer film for lithography, comprising forming an underlayer film on a substrate using the composition for underlayer film formation for lithography according to any one of claims 23 to 25.

**27.** A method for forming a resist pattern, comprising:

forming an underlayer film on a substrate using the composition for underlayer film formation for lithography according to any one of claims 23 to 25;
forming at least one photoresist layer on the underlayer film; and
irradiating a predetermined region of the photoresist layer with radiation for development, thereby forming a resist pattern.

**28.** A method for forming a circuit pattern, comprising:

forming an underlayer film on a substrate using the composition for underlayer film formation for lithography according to any one of claims 23 to 25;

forming an intermediate layer film on the underlayer film using a resist intermediate layer film material having a silicon atom;
forming at least one photoresist layer on the intermediate layer film;
irradiating a predetermined region of the photoresist layer with radiation for development, thereby forming a resist pattern;
etching the intermediate layer film with the resist pattern as a mask, thereby forming an intermediate layer film pattern;
etching the underlayer film with the intermediate layer film pattern as an etching mask, thereby forming an underlayer film pattern; and
etching the substrate with the underlayer film pattern as an etching mask, thereby forming a pattern on the substrate.

**INTERNATIONAL SEARCH REPORT**

International application No.
PCT/JP2018/024048

A. CLASSIFICATION OF SUBJECT MATTER
See extra sheet

According to International Patent Classification (IPC) or to both national classification and IPC

B. FIELDS SEARCHED

Minimum documentation searched (classification system followed by classification symbols)
Int.Cl. G03F7/004, C07D251/24, C09D7/63, C09D201/00, G03F7/023, G03F7/027, G03F7/032, G03F7/039, G03F7/11, G03F7/20, G03F7/26, G03F7/40, H01L21/027

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)
CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| X<br>Y<br>A | JP 2002-356669 A (FUJI PHOTO FILM CO., LTD.) 13 December 2002, claims, examples, paragraphs [0044]-[0050], [0089] (Family: none) | 1-3, 8-13, 15-16<br>14<br>4-7, 17-28 |
| Y | WO 2017/057192 A1 (FUJIFILM CORP.) 06 April 2017, examples, paragraph [0186] & KR 10-2018-0042313 A & TW 201712434 A | 14 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 23 August 2018 (23.08.2018) | 04 September 2018 (04.09.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2018/024048

C (Continuation). DOCUMENTS CONSIDERED TO BE RELEVANT

| Category* | Citation of document, with indication, where appropriate, of the relevant passages | Relevant to claim No. |
|---|---|---|
| X<br><br>A | JP 2016-018093 A (TORAY INDUSTRIES, INC.) 01 February 2016, claims, examples 8, 10-17, comparative examples 1-3, 6-7 (Family: none) | 1-3, 9-13, 16, 22-27<br>4-8, 14-15, 17-21, 28 |
| X<br>A | JP 2010-204661 A (DONGJIN SEMICHEM CO., LTD.) 16 September 2010, claims, example 2 & US 2010/0222473 A1, claims, example 2 & CN 101825843 A & KR 10-2010-0099048 A & TW 201039056 A | 1-3, 8-12, 15-16<br>4-7, 13-14, 17-28 |
| X<br>A | JP 2012-032556 A (FUJIFILM CORP.) 16 February 2012, claims, example 9 & US 2013/0143164 A1, claims, example 9 & WO 2012/015076 A1 & TW 201211696 A & CN 103038706 A & KR 10-2013-0041922 A | 1-3, 8-12, 15-16<br>4-7, 13-14, 17-28 |
| X<br>A | JP 2010-052028 A (FUJIFILM CORP.) 11 March 2010, claims, examples, paragraph [0056] & US 2011/0128522 A1, claims, examples, paragraph [0079] & WO 2010/023790 A1 & EP 2333589 A1 & CN 102132173 A & TW 201009510 A | 1-4, 9-12<br>5-8, 13-28 |
| E, X<br>E, A | JP 2018-116258 A (TOYO INK SC HOLDINGS CO., LTD.) 26 July 2018, claims, example 18 (Family: none) | 1-12, 15-16<br>13-14, 17-28 |

Form PCT/ISA/210 (continuation of second sheet) (January 2015)

**INTERNATIONAL SEARCH REPORT**

International application No.
PCT/JP2018/024048

CLASSIFICATION OF SUBJECT MATTER
G03F7/004(2006.01)i, C07D251/24(2006.01)i, C09D7/63(2018.01)i, C09D201/00(2006.01)i, G03F7/023(2006.01)i, G03F7/027(2006.01)i, G03F7/032(2006.01)i, G03F7/039(2006.01)i, G03F7/11(2006.01)i, G03F7/20(2006.01)i, G03F7/26(2006.01)i, G03F7/40(2006.01)i, H01L21/027(2006.01)i

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2004177668 A
- JP 2004271838 A
- JP 2005250434 A
- WO 2009072465 A
- WO 2011034062 A
- JP 2002334869 A
- WO 2004066377 A
- JP 2007226170 A
- JP 2007226204 A
- JP 2012136520 A
- WO 2013024778 A
- JP 62036663 A
- JP 61226746 A
- JP 61226745 A
- JP 62170950 A
- JP 63034540 A
- JP 7230165 A
- JP 8062834 A
- JP 9054432 A
- JP 9005988 A
- US 5405720 A
- US 5360692 A
- US 5529881 A
- US 5296330 A
- US 5436098 A
- US 5576143 A
- US 5294511 A
- US 5824451 A
- WO 2013024779 A
- WO 2015080240 A
- JP 2017126543 A